# EUROPEAN PATENT SPECIFICATION

(11) **EP 2 705 156 B1**
(45) Date of publication and mention of the grant of the patent: **26.08.2015**
(21) Application number: 12719683.0
(22) Date of filing: 04.05.2012
(51) Int. Cl.: C12Q 1/68

(54) **LINEAR DNA AMPLIFICATION**
LINEARE DNA-AMPLIFIKATION
AMPLIFICATION D'ADN LINÉAIRE

(30) Priority: 05.05.2011 EP 11305531
(43) Date of publication of application: 12.03.2014
(73) Proprietor: Institut National de la Santé et de la Recherche Médicale (INSERM), 75013 Paris (FR); Stichting Dienst Landbouwkundig Onderzoek, 6701 BH Wageningen (NL); Centre National de la Recherche Scientifique (CNRS), 75016 Paris (FR); Université de Strasbourg, 67000 Strasbourg (FR)
(72) Inventor: VERDELHO TRINDADE VAN GERVEN, Luisa Miguel, NL-6708 Wageningen (NL); GRONEMEYER, Hinrich, 67404 Illkirch Cedex (DE); PATTABHIRAMAN, Shankara Narayanan, F-67404 Illkirch Cedex (FR)
(74) Representative: Blot, Philippe Robert Emile
(86) International application number: PCT/EP2012/058194
(87) International publication number: WO 2012/150317

(56) References cited:
- VAN BAKEL HARM ET AL: "Improved genome-wide localization by ChIP-chip using double-round T7 RNA polymerase-based amplification", NUCLEIC ACIDS RESEARCH, vol. 36, no. 4, March 2008 (2008-03), XP002658309, ISSN: 0305-1048
- LIU CHIH LONG ET AL: "Development and validation of a T7 based linear amplification for genomic DNA", BMC GENOMICS, BIOMED CENTRAL, LONDON, GB, vol. 4, no. 1, 19, 9 May 2003 (2003-05-09) , pages 1-11, XP021014452, ISSN: 1471-2164, DOI: 10.1186/1471-2164-4-19
- SHANKARANARAYANAN PATTABHIRAMAN ET AL: "Single-tube linear DNA amplification (LinDA) for robust ChIP-seq", NATURE METHODS, vol. 8, no. 7, July 2011 (2011-07), XP002658319,

## Description

### FIELD OF THE INVENTION

The present invention concerns materials and methods for DNA amplification, in particular linear amplification methods using RNA polymerase. These methods permit high-throughput sequencing of picogram amounts of DNA and are of potential use in a range of applications, including genome-wide profiling of transcription factors and epigenetic DNA and histone modifications, global transcript profiling, mapping of chromatin conformations, as well as for forensic use and archaeological studies. The methods of the invention may be carried out in a single reaction vessel, reducing DNA loss and making the procedure suitable for automation.

### BACKGROUND

Analysis of DNA present in very limited amounts, for example in forensics, archaeological studies or small cell samples, requires the DNA to be amplified prior to analysis. Many techniques of DNA amplification have been developed, most notably the polymerase chain reaction (PCR). However, PCR is an exponential amplification method and is thus not ideally suited for quantitative analysis. Exponential methods of DNA amplification are liable to bias, because sequence- or length-dependent biases in the amplification are themselves exponentially amplified. For example, PCR is known to favour amplification of GC-rich sequences, and will thus overestimate the prevalence of such sequences. Linear DNA amplification methods using T7 RNA polymerase have been reported, but all require relatively large (nanogram) amounts of starting DNA and are not compatible with high-throughput sequencing or automation.

One area where a reliable and consistent method of DNA amplification is required is the study of epigenetic transcription control programs and chromatin conformation. Technologies such as chromatin immunoprecipitation (ChIP), chromatin interaction analysis by paired-end tag sequencing (ChIA-PET) and Hi-C, coupled with high throughput sequencing (HTS), can be used to provide a genome-wide view of chromatin modification, conformation and dynamics and the action of transcription modulating complexes. However, the need to recover nanogram amounts of immunoprecipitated DNA represents a serious limitation for the use of such techniques. This is a particular problem for analysis of certain cell types of major (patho)physiological importance, which may be available in very limited numbers, such as stem cells, cancer initiating cells or specific groups of cells during early development and organogenesis. ChIP studies of chromatin histone modifications for small cell numbers have been performed, but the techniques used are not compatible with HTS. To carry out genome-wide sequencing, it is therefore necessary to pre-amplify ChIPed DNA before sequencing. As explained above, current DNA amplification protocols are not suitable for such procedures, as the requirement for multiple ligations and exponential amplification make them prone to the introduction of artefacts and amplification bias. As a consequence, ChIPs using antibodies directed against modified histones yield comparatively high recoveries of immunoprecipitated DNA and generate profiles that frequently present broad peaks, if compared with transcription factor (TF) profiling. To date, no versatile technique has been described that (i) demonstrates reliable amplification of picogram DNA quantities of complex DNA samples corresponding to TF binding sites to chromatin and (ii). can be used for HTS or the analysis of forensic or archaeological specimens from which only ultra-small amounts of DNA can be recovered.

Non-exponential DNA amplification techniques using T7 RNA polymerase-based amplification have been developed, and their fidelity and non-biased nature demonstrated (see C. L. Liu, S. L. Schreiber, and B. E. Bernstein, BMC Genomics 4 (1), 19 (2003); H. van Bakel, F. J. van Werven, M. Radonjic et al., Nucleic Acids Res 36 (4), e21 (2008); Chih Long Liu, Bradley E. Bernstein, and Stuart L. Schreiber, Cold Spring Harb Protoc 2008 (5), pdb.top42 (2008)). However, the products of these reactions cannot be directly used for HTS. Furthermore, these protocols are incompatible with ultra-small amounts of DNA, as they involve complex handling steps involving column purifications with the inherent risks of sample losses and cross contaminations.

A DNA amplification method suitable for HTS has been reported (Adli et al, 2010, Nat Methods 7, 615), but the method is PCR based and thus subject to all of the disadvantages arising from use of this technique, in particular amplification bias of GC-rich sequences.

There is thus a need in the art for a method of linear amplification of DNA that can be used with sub-nanogram starting quantities of DNA. A method that is compatible with high throughput sequencing would be of particular utility.

### SUMMARY OF THE INVENTION

To address this need, the present inventors have developed a novel single-tube RNA polymerase-based linear DNA amplification strategy, which they have shown can amplify DNA obtained from TF ChIPs as low as 30 pg with high fidelity and simplicity. This technology, referred to as LinDA (**Lin**ear **D**NA **A**mplification) is compatible with HTS. The inventors have also developed a single buffer that can be used for multiple steps of the method. Consecutive steps are performed in the same tube by sequential addition of reagents, thus eliminating the need for column purification and minimizing the risk of sample losses particularly at early steps. This feature makes it suited for process automation using a liquid handling machine.

The inventors have demonstrated that LinDA can be used for single-tube ChIP-seq and re-ChIP-seq with picogram DNA amounts obtained from a few thousand cells. They have also successfully used the procedure for PAT-ChIP-seq using DNA samples from paraffin-embedded sections. LinDA-generated ChIP-seq profiles are comparable to biological replicates. This amplification technology has many potential applications. For example, it can facilitate global TF binding and chromatin analyses with very small cell populations, such as stem or cancer initiating cells. In addition it will be generally useful for forensic use and archaeological sciences. Finally, the single tube concept will enable LinDA automation which will greatly enhance the efficiency and cost-effectiveness of the procedure.

Thus, one aspect of the present invention relates to a method of linear DNA amplification comprising the steps:
(i) T-tailing DNA ends of double-stranded DNA fragments in a sample;
(ii) annealing to said fragments primers comprising an RNA polymerase promoter site upstream of a poly-A tail, wherein said primers anneal to the poly-T ends of said fragments;
(iii) using a 5'-3' DNA polymerase to synthesise DNA complementary to the primer overhangs, to create double-stranded DNA fragments with an RNA polymerase promoter site at both ends;
(iv) in vitro transcribing said DNA using an RNA polymerase which binds to said RNA polymerase promoter site;
(v) reverse transcribing the RNA products of step (iv) to create single-stranded DNA products;
(vi) creating double stranded DNA fragments by second strand synthesis of the single-stranded DNA of step (v);
(vii) optionally, repeating steps (iv)-(vi).

In one embodiment; the method of the invention comprises the steps:
(i) incubating the double-stranded DNA sample with alkaline phosphatase in order to dephosphorylate 3' ends;
(ii) inactivation of alkaline phosphatase by heat treatment of the sample;
(iii) incubating the sample with terminal transferase and dTTPs for T-tailing of DNA ends;
(iv) inactivation of terminal transferase by heat treatment of the sample;
(v) adding to the sample primers comprising an RNA polymerase promoter site upstream of a poly-A tail and incubating to allow annealing of the primers to the sample DNA;
(vi) adding to the sample a 5'-3' DNA polymerase and dNTPs and incubating at 37 °C;
(vii) inactivation of DNA polymerase by heat treatment of the sample;
(viii) adding to the sample an RNA polymerase which binds to said RNA polymerase promoter site, NTPs and the primer of step (v), and incubating to allow in vitro transcription of said DNA;
(ix) adding to the sample a reverse transcriptase, an RNAse, dNTPs and the primer of step (v) and incubating to allow reverse transcribing the RNA products of step (viii) to create single-stranded DNA products;
(x) adding to the sample an RNAse, a DNA polymerase and dNTPs for Second strand synthesis of the single-stranded DNA of step (ix); (xi) optionally, repeating steps (yiii)-(x).

The in vitro transcription step may be followed by a step of extraction or separation of RNA from the sample prior to the reverse transcription step.

In some embodiments, said primer further comprises a restriction enzyme cleavage site downstream of the RNA polymerase promoter site sequence. The method may further comprise the step of removing the primers from the DNA ends by digestion with a restriction enzyme that recognized said restriction enzyme cleavage site. In a preferred embodiment, the primer comprises a restriction enzyme cleavage site downstream of the RNA polymerase promoter site and upstream of the poly A tail, wherein said restriction enzyme site is optionally a Bpm1 site, and wherein said poly A tail is optionally 15 or 16 nucleotides in length.

In one embodiment; the method of the invention comprises the steps:
(i) incubating the double-stranded DNA sample with alkaline phosphatase in order to dephosphorylate 3' ends;
(ii) inactivation of alkaline phosphatase by heat treatment of the sample;
(iii) incubating the sample with terminal transferase and dTTPs for T-tailing of DNA ends;
(iv) inactivation of terminal transferase by heat treatment of the sample;
(v) adding to the sample primers comprising an RNA polymerase promoter site upstream of a poly-A tail and incubating to allow annealing of the primers to the sample DNA;
(vi) adding to the sample a 5'-3' DNA polymerase and dNTPs and incubating at 37°C;
(vii) inactivation of DNA polymerase by heat treatment of the sample;
(viii) adding to the sample an RNA polymerase which binds to said RNA polymerase promoter site, NTPs and the primer of step (v), and incubating to allow in vitro transcription of said DNA;
(ix) adding to the sample a reverse transcriptase, an RNAse, dNTPs and a first sequencing adapter primer, and incubating to allow reverse transcribing the RNA products of step (viii) to create single-stranded DNA products;
(x) removing excess said first sequencing adapter primer;
(xi) adding to the sample an RNAse, a DNA polymerase, a second sequencing adapter primer and dNTPs for second strand synthesis of the single-stranded DNA of step (ix);
(xi) optionally, repeating steps (viii)-(x).
Also provided is a method of making a sequencing library, said method comprising steps (i)-(xi) above. A sequencing library produced by said method is also provided.

Said sequencing adapter primers may be, for example, said first and second sequencing adapter primers may be the flowcell and bridge adapters from the Illumina (Solexa), sequencing technology, or vice versa, or their equivalent in the Roche (454), SOLiD or Ion torrent sequencing technology.

Removal of excess first sequencing adapter primer at step (x) may be achieved by incubation with a DNA exonuclease, such as Exo1.

The enzymes used in the various steps of the method are routinely used in molecular biology and suitable enzymes will be well known to the skilled person. Examples of said enzymes are discussed below. For example, said 5'-3' DNA polymerase used to synthesise DNA complementary to the primer overhangs may be a Klenow polymerase, said RNA polymerase may be a T7 RNA polymerase, said reverse transcription an AMV reverse transcriptase and said DNA polymerase used for second strand synthesis aTaq and/or a Pfu polymerase.

Heat treatment should be sufficient to denature the enzyme in the sample. The degree and duration of the treatment can be easily determined by the skilled person, as the denaturation temperature of commercially available enzymes is known.

Preferably, the heat treatment is performed at a temperature that does not denature the DNA in the sample. This is particularly important at the stage of using a 5'-3' DNA polymerase to synthesise DNA complementary to the primer overhangs, as it is the lack of denaturation of the strands before end filling which permits the creation of double-stranded DNA fragments with an RNA polymerase promoter site at both ends.

Suitable heat treatment may comprise heating to between 65 and 75°C, for example to 65 °C , 66°C, 67°C, 68°C, 69°C, 70°C 71°C, 72°C, 73°C, 74°C or 75°C for a period of at least 1, 2, 3, 4, 5, 6, 7, 8, 9, 10, 15, 20, 30, 60 minutes or more, or overnight. For example, heat treatment may comprise heating the sample to 72°C for 10 minutes.

Incubation of a sample with enzyme involves maintaining the sample at a temperature compatible with enzyme activity for an appropriate period. Incubation temperatures for most enzymes are between 20 and 47°C, depending on the source organism of the enzyme, for example 20, 21, 22, 23, 24, 25, 26, 27, 28, 29, 30, 31, 32, 33, 34, 35, 36, 37, 38, 39, 40, 41 42, 43, 44, 45, 46 or 47°C. For most of the enzymes used in method of the present invention, incubation is at or around 37°C, though certain enzymes such as reverse transcriptases function most efficiently at a higher temperature, preferably at or around 42°C. Incubation may be carried out for e.g. 1, 2, 3, 4, 5, 6, 7, 8, 9, 10, 15, 20, 30, 60 minutes or more, or overnight. Certain enzymes, such as terminal transferase, should be incubated for shorter periods, for example 20 minutes. The optimal temperature and period of incubation can be readily determined by the skilled person based on the known properties of these enzymes.

In a preferred embodiment, one or more of the steps of said method are carried out in a buffer comprising 20mM Tris-acetate, 10mM magnesium acetate, 50mM potassium acetate, and 1 mM dithiothreitol at pH 7.9, or an equivalent buffer as discussed below. In a particularly preferred embodiment, all of the steps of the method up to and including the in vitro transcription step are carried out in said buffer.

In some embodiments, the method further comprises sequencing of the amplified DNA fragments, in particular high-throughput sequencing.

As discussed above, the method of the invention is of particular use in amplifying DNA samples obtained by chromatin immunoprecipitation embodiments, therefore, the starting sample of double stranded DNA fragments is obtained by ChIP, reCHiP or PAT-ChIP. The sample may also be obtained by ChIA-PET or Hi-C.

In certain embodiments, multiple steps of the claimed method are carried out in a single reaction vessel. For example, all of the steps may be carried out in the same vessel. Alternatively, for example where the reverse transcriptase step is followed by RNA extraction or separation, all of the steps up to and including the reverse transcriptase step may be carried out in the same vessel. This has the advantage that the steps are carried out in the same vessel, for example a single tube or microwell plate, without the need to transfer the sample between vessels or apply it to columns, all of which processes risk losing DNA. These steps may thus be automated, which is of particular use in large scale analysis, for example large scale analyses of patients to determine thei epigenetics profiles.

In a particular embodiment, the method comprises the steps
(i) incubating the double-stranded DNA sample with alkaline phosphatase at 37°C in order to dephosphorylate 3' ends;
(ii) inactivation of alkaline phosphatase by heat treatment of the sample;
(iii) incubating the sample with terminal transferase and dTTPs at 37°C for T-tailing of DNA ends;
(iv) inactivation of terminal transferase by heat treatment of the sample;
(v) adding to the sample primers comprising an T7 RNA polymerase promoter site upstream of a poly-A tail, and incubating at 37°C to allow annealing of the primers to the sample DNA;
(vi) adding to the sample Klenow polymerase and dNTPs and incubating at 37°C;
(vii) inactivation of Klenow polymerase by heat treatment of the sample;
(viii) in vitro transcribing said DNA using T7 RNA polymerase;
(ix) extracting the RNA products of step (iv);
(x) reverse transcribing the RNA products of step (iv) to create single-stranded DNA products;
(xi) incubating with Taq polymerase, Pfu polymerase and RNAse H at 37°C to creating double stranded DNA fragments by second strand synthesis of the single-stranded DNA of step (ix) ;
(xii) optionally, repeating steps (viii)-(xi);
wherein steps (i) - (viii) of said method are carried out in a buffer comprising 20mM Tris-acetate, 10mM magnesium acetate, 50mM potassium acetate, 1 mM dithiothreitol at pH 7.9, and optionally wherein steps (i) - (viii) of said method are carried out in a single reaction vessel.

The methods of the invention may also be used to analyse RNA samples. In such a case, the method would be preceded by the additional steps of transcribing the RNA to create cDNA, followed by second strand synthesis to create double-stranded DNA.

### Buffers and reaction mixtures

The term 'buffer', as used herein, refers to a solution containing a buffering agent or a mixture of buffering agents and, optionally, a divalent cation and a monovalent cation.

The term 'reaction mixture', as used herein, refers to an aqueous solution comprising the various reagents used for a given enzymatic reaction. These may include enzymes, aqueous buffers, salts, amplification primers, target nucleic acid, and nucleoside triphosphates (NTPs) or deoxyribonucleoside triphosphates (dNTPs). Depending upon the context, the mixture can be either a complete or incomplete amplification reaction mixture. For example, the mixture may contain all the buffering elements required for enzymatic activity, but lack certain enzymes or dNTPs.

In a preferred embodiment of the invention, the buffer or reaction mixture is compatible with more than one, or all, of the enzymatic reactions which form a part of the method of the invention, namely alkaline phosphatase, terminal transferase, DNA polymerase and/or reverse transcriptase.

The monovalent cation is typically supplied by the potassium, sodium, ammonium, or lithium salts of either chloride or acetate. The concentration monovalent cation is typically between 1 and 200 mM, preferably between 40 and 100 mM.

DNA polymerases and terminal transferases require a divalent cation for catalytic activity. For extension reactions using a DNA template, the preferred divalent cation is Mg²⁺, although other cations, such as Mn²⁺ or Co²⁺ can activate DNA polymerases. For terminal transferase activity, Co²⁺ is preferred, though Mg²⁺ and Mn²⁺ can also be used. For reverse transcription using a thermostable polymerase, Mn²⁺ is preferred as the divalent cation. The divalent cation is typically included as a salt, for example a chloride, acetate or sulphate salt, e.g. MgCl₂, MgCl₂, Mg(OAc)₂, MgSO₄, MnCl₂, Mn(OAc)₂, or MnSO₄. In general, for reactions using Mn⁺², usable cation concentrations in a Tris buffer will be in a range from 0.5 to 7 mM, preferably between 0.5 and 2 mM. In general, for reactions using Mg⁺², usable divalent cation concentrations in a Tris buffer will be in a range from 0.5 to 10mM MgCl₂.

A buffer solution may also contain a reducing agent, such as dithiothreitol or mercaptoethanol.

In a preferred embodiment of the invention, the buffer or reaction mixture is compatible with all of the enzymatic reactions which form a part of the method of the invention, namely alkaline phosphatase, terminal transferase, DNA polymerase and/or reverse transcriptase. The inventors have succeeded in developing a buffer which fulfils these requirements and thus allows all of the method steps to be carried out in the same solution, avoiding the need for column purifications and transfer of nucleic acid between reaction vessels.

Thus, a preferred buffer solutions for use in the method of the present invention comprises Tris at 5-50mM, for example at or around 5, 10, 15, 20, 25, 30, 35, 40, 45 or 50mM, a Mg²⁺ salt at 5 to 15mM, for example at or around 5, 6, 7, 8, 9, 10, 11, 12, 13, 14, or 15mM, a potassium or sodium salt at 25 to 75mM, for example at or around 25, 30, 35, 40, 45, 50, 55, 60, 65, 70 or 75mM, and a reducing agent such as dithiothreitol at 0.5 to 5mM, for example at or around 0.5, 1, 1.5, 2, 2.5, 3, 3.5, 4, 4.5, or 5 mM. A pH range of 7.5 to 8.5, for example at or around 7.5, 7.6, 7.7, 7.8, 7.9, 8.0, 8.1, 8.2, 8.3, 8.4 or 8.5 is preferred. A particularly preferred solution comprises 20mM Tris-acetate, 10mM magnesium acetate, 50mM potassium acetate and 1 mM dithiothreitol at pH 7.9 Specific buffers may amso be used. For example, a reverse transcriptase buffer may be: 50 mM Tris HCl (pH 8.3), 75 mM KCI, 3 mM MgCl2, 20 mM DTT. For second strand synthesis, an exemplary buffer may be: 20 mM Tris HCl (pH 8.8), 10 mM (NH4)SO4, 10 mM KCl, 2 mM MgSO4, 0.1% Triton X100, 0.1 mg/ml BSA. For in vitro transcription, an exemplary reaction solution may be: 1 x RNAmaxx transcription buffer (STRATAGENE), 4 mM of each rUTP, rGTP, rATP, rCTP; 0.03M DTT, 0.5 µl 0.75 U/µl yeast inorganic pyrophosphatase, 1 µl RNaseblock, 1 µl of 200U/µl T7 RNA polymerase

The skilled person will understand that minor variations in a given buffer concentration may be introduced without affecting the efficacy of the reaction. For example, a variation of 1, 2, 3, 4, 5, 6, 7, 8, 9, 10, 15, 20, 25% or more may be tolerated.

The enzymatic reactions carried out in the method of the present invention are widely used in molecular biology. The skilled person can easily determine appropriate concentrations of enzyme and additional reagents, such as NTPs or dNTPs, required for the reactions. Generally, the concentration of dNTPs in an amplification reaction using a Tris buffer is around 200nM for each dNTP. Enzyme amounts are typically in the range of 1 to 10 units per reaction or according to the manufacturer's instructions.

### Primers

The term "primer" as used herein refers to an oligonucleotide primer, whether natural or synthetic, which is capable of acting as a point of initiation of nucleic acid synthesis when placed under conditions in which primer extension (not limited in number of extended bases) is initiated. A primer is preferably a single-stranded oligodeoxyribonucleotide. The appropriate length of a primer for use in the present invention is, as appreciated in the relevant art, depends on the intended use of the primer but typically ranges from about 30 to about 50 nucleotides, for example 30, 31, 32, 33, 34, 35, 36, 37, 38, 39, 40, 41, 42, 43, 44, 45, 46, 47, 48, 49, or about 50 nucleotides. Short primer molecules generally require cooler temperatures to form sufficiently stable hybrid complexes with the template. A primer need not reflect the exact sequence of the template but must be sufficiently complementary to hybridize with a template for primer elongation to occur. A primer can be labeled, if desired, by incorporating a label that is detectable by, for example, spectroscopic, photochemical, biochemical, immunochemical, or chemical means. Exemplary labels include, but are not limited to radiolabels (e.g., ³²P), fluorescent dyes, electron-dense reagents, enzymes (as commonly used in ELISAS), biotin, or haptens and proteins for which antisera or monoclonal antibodies are available.

Preferred primers for use in the method of the invention comprise a binding site for an RNA polymerase and a poly-A tail. The poly-A tail is preferably at least 5, at least 10, at least 15 or at least 20 nucleotides in length. The RNA polymerase binding site preferably includes a promoter sequence, i.e. a nucleic acid sequence that regulates expression of a transcriptional unit. A "promoter region" is a regulatory region capable of binding RNA polymerase in a cell and initiating transcription of a downstream (3' direction) coding sequence. Within the promoter region will be found a transcription initiation site (conveniently defined by mapping with nuclease S1), as well as protein binding domains (consensus sequences) responsible for the binding of RNA polymerase such as the putative -35 region and the Pribnow box. Suitable promoter regions are discussed below.

It may be desirable to include a restriction enzyme recognition sequence in the primer, preferably downstream (3') of the RNA polymerase promoter site. This permits the primer sequence to be cleaved from the ends of the DNA fragments after amplification, which is particularly useful when further analysis of the DNA is to be performed, for example sequencing. To permit optimal ligation of the poly-A tail to the poly-dT tails of the DNA fragments, a restriction enzyme that cuts downstream of its recognition sequence may be used. In such a case, the primer will comprise an RNA polymerase binding site at its 5' end, a restriction enzyme recognition sequence downstream of the RNA polymerase binding site, and a poly A tail at its 3' end. The length of the poly A tail may then be designed so that the restriction enzyme cleavage site falls at the end of the poly A tail. For example, the restriction enzyme Bpm1 cleaves DNA at a site 16 base pairs downstream of its recognition sequence. A primer comprising an RNA polymerase binding site and the Bpm1 recognition site CTGGAG followed by (dA)₁₆ could thus be used, which would allow the primer sequence to be cleanly cleaved from the ends of the DNA fragments by Bpm1 after amplification. Other type II enzymes such as Mmel, Eco p151, Fokl, Acul, Aarl, Alol, AsiSl, Ppl, Psrl, Bael, BsaXI, Bmrl, Bcgl, BpuEl, BspCNI, BseR1, Bbvl, Faul, Ecil and Bsal may be used in a similar way. These enzymes, their recognition and cleavage sites are all well known in the art and suitable primers may be readily designed by the skilled person.

In some embodiments of the invention, primers used in the library construction of commercially available sequencing technology may be incorporated into the procedure, as described above. For example, the primers used in the Illumina (Solexa) sequencing technology (True Seq DNA sample preparation kit V2, Ilumina, catalogue no FC-121-2001) may be used, as may their equivalent in the Roche (454), SOLiD or Ion torrent sequencing technology (NEB catalogue numbers as follows: Illumina: E6000S/L; 454 (Roche) : E6080S/L; SOLiD: E6060S/L; Ion Torrent: E6270S/L)

### Terminal transferase and T-tailing

'T-tailing' as used herein refers to the procedure of attaching a dNTP or dNTPs to the 3' end of a DNA strand. Where the DNA strand is part of a DNA duplex, a T-tailing reaction will result in double-stranded DNA with a T or poly-T overhang at each end.

T-tailing is carried out using a terminal transferase enzyme, which catalyses the addition of nucleotides to the 3' terminus of DNA. Unlike most DNA polymerases it does not require a template. The preferred substrate of this enzyme is a 3'-overhang, but it can also add nucleotides to blunt or recessed 3' ends. Cobalt is a necessary cofactor *in vivo*, though the enzyme can catalyze reactions upon Mg and Mn administration *in vitro*. Usually, the terminal transferase reaction is preceded by a step of dephosphorylating the DNA ends using an alkaline phosphatase enzyme. Preferably, the alkaline phosphatase is one which can be inactivated by heat treatment, for example shrimp alkaline phosphatase.

### RNA polymerase

RNA polymerases produce RNA using DNA as a template, by polymerising ribonucleotides at the 3' end of an RNA transcript.

The RNA polymerase most widely used in the production of recombinant proteins is the viral RNA polymerase RNA polymerase encoded by bacteriophage T7. Mutants of T7RNA polymerase are also known, and have been developed specifically to enhance *in vitro* production of RNA (see for example Makarova, et al. (1995) Proc Natl Acad Sci U S A 92:12250-4).

Sequence requirements for RNA polymerase binding sites are well known in the art. Various T7 RNA polymerase promoter sequences are known, including natural sequences (Dunn & Studier (1983) J Mol Biol 166:477-535) and artificial sequences (e.g. see refs. 21-26). Different T7 RNA polymerases can have different promoter sequence preferences, and mutant T7 RNA polymerases have been produced to match specific promoters. These sequences are all well known and routinely used in the art, and the skilled person can easily obtain both T7 RNA polymerases and promoter sequences and match any particular T7 RNA polymerase to its preferred promoter sequence.

The consensus 23 base-pair T7 DNA promoter is classically divided into two domains, an upstream binding domain (-17 to -5, numbered relative to the start of transcription), and a downstream initiation domain (-4 to +6). This 23mer is: TAATACGACTCACTATAGGGAGA (SEQ ID NO: 1). The minimum sequence required for efficient transcription is the first 19mer of SEQ ID NO: 1, ie TAATACGACTCACTATAGG. Thus, where the T7 RNA polymerase is used in the method of the invention of the invention, the primers will preferably include at least the 19-mer and preferably the 21-mer.

Other RNA polymerases routinely used *in vitro* include the SP6 and T3 polymerases. An SP6 promoter site may comprise ATTTAGGTGACACTATAG (SEQ ID No 2). A T3 promoter site may comprise ATTAACCCTCACTAAAGGGA (SEQ ID No 3).

Preferred RNA polymerases are those which are able to traverse template discontinuity, in particular nicks and gaps, in the template strand of double-stranded DNA. Such polymerases include T7 and SP6 polymerases. The use of any suitable RNA polymerase is nonetheless included within the scope of the present invention. The skilled person will select the polymerase and promoter site according to the reagents available in the art at the time.

### DNA polymerase

DNA polymerase catalyses the polymerization of deoxyribonucleotides into a DNA strand, using an existing polynucleotide strand as template. DNA polymerase can add free nucleotides to only the 3' end of the newly-forming strand, resulting in elongation of the new strand in a 5'-3' direction. It can only add a nucleotide onto only a preexisting 3'-OH group, and, therefore needs a DNA or RNA primer in order to initiate synthesis.

Some DNA polymerases also have 3'-5' exonuclease activity, i.e; the ability to remove nucleotides by catalysis of the hydrolysis of the phosphodiester bond. This permits them to correct mistakes in newly-synthesized DNA. When an incorrect base pair is recognized, DNA polymerase reverses its direction by one base pair of DNA. The 3'-5' exonuclease activity of the enzyme allows the incorrect base pair to be excised (this activity is known as *proofreading*). Following base excision, the polymerase can re-insert the correct base and replication can continue. Certain DNA polymerases, such as polymerase I, also have a 5'-3' exonuclease activity, i.e; they can also remove nucleotides in the 5'-3' direction.

Klenow polymerase, or the Klenow fragment, is a fragment of the DNA polymerase I from *E. coli* which retains the 5'-3' polymerase activity and the 3' → 5' exonuclease activity for removal of precoding nucleotides and proofreading, but has no 5' → 3' exonuclease activity. The Klenow fragment was first described in1970 (Klenow and Henningsen (1970) Proc Natl Acad Sci 65 (1): 168-175), and since then has been widely used in molecular biology for procedures such as synthesis of double-stranded DNA from single-stranded templates, filling in recessed 3' ends of DNA fragments to create blunt ends, and digesting away protruding 3' overhangs. It has a preference for gaps (Xu et al (2000) JBC 275, 20949-20955) and is thus ideally suited to gap filling. Klenow polymerase is thus suited for the step using a 5'-3' DNA polymerase to synthesise DNA complementary to the primer overhangs to create double-stranded DNA fragments with an RNA polymerase promoter site at both ends. Other enzymes which may be used for this step include EcoPol I, which also prefers gaps, and Taq polymerase. Alternative DNA polymerases includeT7, T4 DNA pol, phusion and pfu polymerases

### Reverse transcriptase

Reverse transcriptases, also known as RNA-dependent DNA polymerase, are DNA polymerases that uses RNA as a template. All known reverse transcriptases require a primer to synthesize a DNA transcript from an RNA template. Historically, reverse transcriptase has been used primarily to transcribe mRNA into cDNA which can then be cloned into a vector for further manipulation.The most well-studied and frequently used reverse transcriptases include HIV-1 reverse transcriptase from human immunodeficiency virus type 1 (PDB 1 HMV), M-MLV reverse transcriptase from the Moloney murine leukaemia virus, AMV reverse transcriptase from the avian myeloblastosis virus, and the eukaryotic telomerase reverse transcriptase. AMV reverse transcriptase is preferred for use in the invention.

Although 'DNA polymerase' is the term usually used for the class of DNA polymerases characterized as DNA-dependent DNA polymerases, i.e requiring a DNA template for synthesis of DNA, some DNA polymerases are also capable of *in vitro* reverse transcription of RNA, including E coli pol 1 (Gulati et al. 1974, Proc. Nat. Acad. Sci. USA 71:1035-1039) found that *E. coli* Pol I could be used to transcribe Qβ viral RNA using oligo(dT).

The reverse transcriptase step of the invention may be carried out using the same primer as that used for the RNA polymerase step, as discussed above. Existing primer present in the sample from previous steps may be used. Alternatively, fresh primer may be added, in particular if RNA is extracted prior to reverse transcriptase.

### 2^{nd} strand synthesis

As used herein 'second strand synthesis' refers to the synthesis of the complementary DNA strand from an existing single-stranded DNA or DNA-RNA hybrid. Where a DNA-RNA hybrid is the template, as for example when the product of a reverse transcription reaction is used as template, the RNA will need to be removed by digestion prior to second strand synthesis. For example, an RNAse such as RNAse H may be used to nick the DNA/RNA hybrid, and a DNA polymerase used to catalyse the second strand cDNA synthesis using the RNA fragments as primers.

Where there is no primer at the very end present for DNA synthesis, this produces a second strand DNA copy that lacks the last 5-20 base pairs at the 5' termini. The reaction may then be treated with DNA ligase to ligate all of the pieces of DNA that make up the second strand, since second strand synthesis starts at multiple locations from random RNA primers left following RNaseH treatment. Finally, T4 DNA polymerase, Pfu polymerase or other polymerase having 3-5' exonuclease activity may be added to polish the 3' end of the first strand.

In a preferred embodiment of the invention, the primers containing an RNA pomymerase binding site and a poly-T tail are also present during the second strand synthesis step, so there is no requirement for the polishing step. Preferred enzymes for second strand synthesis using this method include EcoPol I and Taq polymerase. Alternatively, other DNA polymerases may be used, for example Klenow, T7, T4 DNA pol, phusion and pfu polymerases.

### ChIP

Chromatin Immunoprecipitation (ChIP) is used to investigate the interaction between proteins and DNA in the cell. It aims to determine whether specific proteins are associated with specific genomic regions, such as transcription factors on promoters or other DNA binding sites, and possibly defining cistromes. CHIP can also be used to determine the specific location in the genome with which various histone modifications are associated, indicating the target of the histone modifiers. In the ChiP technique, protein and associated chromatin in a cell lysate are temporarily bonded, the DNA-protein complexes (chromatin-protein) are then sheared and DNA fragments associated with the protein(s) of interest are selectively immunoprecipitated, and the associated DNA fragments are purified and their sequence is determined. These DNA sequences are supposed to be associated with the protein of interest *in vivo*.

### ChIA-PET and Hi-C

Chromatin interaction analysis by paired-end tag sequencing (ChIA-PET) Hi-C (Genome-wide chromosome conformation capture) are used to analyse long-range chromatin interactions and the three-dimensional conformation of chromosomes. These procedures result in the isolation, by IP or biotin pull-down, of ultra-small amounts of DNA if starting from reasonable amounts of crosslinked cells (20-100 million cells). The DNA targeted by these procedures corresponds to DNA strands that are held in proximity by architectural proteins (APs) and/or transcription factors (TFs) that generate chromatin "loops".

The basic procedure of ChIA-PET and Hi-C is first to generate two dsDNA fragments that correspond to the base of chromatin loops tethered together by APs and/or TFs. For Hi-C the ends of the DNA fragments are repaired and biotin is incorporated; for ChIA-PET linkers are attached. The next step involves ligation under conditions that favour intra-molecular reactions which aims at covalently linking the separate tethered DNA fragments. This is followed by de-crosslinking and digestion. Another set of primers is attached and PCR is performed to amplify the material for sequencing.

The PCR amplification step may be replaced by the method of the invention, in order to avoid the known disadvantages of PCR, in particular GC-rich amplification bias in favour of GC-rich sequences. This would improve the existing method significantly, by reducing the number of cells required, and by increasing the fidelity of amplification.

### High-throughput sequencing

Genome-wide analyses frequently involve sequencing on a large scale. Great efforts have been made to increase the speed and efficiency and cost-effectiveness of sequencing, and there are now many techniques available for high-throughput and 'massively parallel' sequencing methods which permit the simultaneous sequencing of thousands or millions of DNA sequences in a highly automated procedure (see for example Rogers and Venter, Nature 2005 437, 326-327).

Some examples of commercially available high-throughput sequencing methods include Lynx Therapeutics' Massively Parallel Signature Sequencing (MPSS), developed in the 1990s at Lynx Therapeutics; Polony sequencing, now incorporated into the Applied Biosystems SOLiD platform; 454 pyrosequencing, developed by 454 Life Sciences and now acquired by Roche Diagnostics; Illumina (Solexa) sequencing, Applied Biosystems' SOLiD technology.

LinDA can be used to directly generate sequencing libraries by incorporating the sequencing adapters into the procedure, as described below.

A detailed description of one example of a LinDA amplification protocol provided by the invention is given below.

### Reagents

Shrimp alkaline phosphatase (1 U/µl; Promega, cat. no. M820A)
Terminal Transferase (20 U/µl; New England Biolabs, cat. no. M0252S)
RNAMaxx high yield kit (Stratagene, cat. no. 200339; containing 5x transcription buffer, 1 mM of rATP, rCTP, rGTP and rUTP, 0.75 M DTT, yeast pyrophosphatase, RNAse inhibitor, T7 RNA polymerase).
Klenow fragment (10 U/µl; New England Biolabs, cat. no. M0210S)
Superscript III reverse transcription kit (Invitrogen, cat. no. 18080-044 or 18080-085; containing 5x reaction buffer, 0.1 M DTT, AMV reverse transcriptase 200U/µl.
RNAse H (5 U/µl; New England Biolabs, cat. no. M0297S)
Taq polymerase (5 U/µl; Roche, cat. no. 11435094001)
Pfu polymerase (5 U/µl; Stratagene, cat. no. 600159)
Bpm I (2.5 U/µl; New England Biolabs, cat. no. R0565S)
RNasin Plus RNase inhibitor (Promega, cat. no. N2611 or N2615)
dNTP mix (10 mM mix of dATP, dTTP, dCTP and dGTP; GE Healthcare, cat. no. 28-4065-64)
ddCTP (100 mM; GE Healthcare, cat. no. 27-2061-01)
dTTP (100 mM; GE Healthcare, cat. no. 28-4065-31)
NEB buffer 4 (New England Biolabs, cat. no. B7004S; 1x comprises of 20 mM Tris-acetate pH 7.9, 10 mM magnesium acetate, 50 mM potassium acetate, 1 mM DTT)
Thermopol buffer (New England Biolabs, cat. no. B9004S; 1 x comprises of 20 mM Tris-HCl pH 8.8, 10 mM KCl, 10 mM ammonium sulphate, 2 mM magnesium sulphate, 0.1% Triton X-100)
BSA (100x; New England Biolabs, cat. no. B9001S)
QIAquick PCR purification kit (50 columns; Qiagen, cat. no. 28104)
MinElute PCR purification kit (50 columns; Qiagen, cat. no. 28004)
GenElute mammalian total RNA miniprep kit (70 columns; Sigma, cat. no. RTN70)

### Procedure

### Steps 1-4: Dephosphorylation

Set up 17 µl reaction for each DNA sample in a 200 µl PCR tube as follows

| **Component** | **Volume (µl)** |
|---|---|
| ChIP DNA | 14 |
| NEB 4 Buffer | 2 |
| Shrimp alkaline phosphatase (1 U/µl) | 1 |
| Total | 17 |

Place the tube in PCR machine with a heated lid and incubate at 37 °C for 10 min Inactivate the enzyme by incubating at 70 °C for 10 min

### Cool to 4 °C

The reaction is performed in a 200 µl PCR tube using a PCR machine to perform the different steps at the indicated temperatures. This simplifies the procedure as the successive steps can be performed in the same tube with the addition of the different reagents.
Dephosphorylation improves the efficiency of the terminal transferase reaction.
The DNA reaction can be stored at -20 °C for at least 1 year.

### Steps 5-8: 'T' tailing

Add the following components to the tube from the previous step

| **Component** | **Volume (µl)** |
|---|---|
| DNA reaction from previous step | 17 |
| T mix | 1 |
| 5 mM CoCl₂ | 1 |
| Terminal transferase (20U/µl) | 1 |
| Total | 20 |

Incubate for 20 min at 37 °C.
Inactivate the enzyme at 70 C for 10 min.
Cool to 4 °C.
The DNA reaction can be stored at -20 °C for at least 1 year.

### Steps 6-14: Primer annealing and extension

Add the following components to the tube from the previous step

| **Component** | **Volume (µl)** |
|---|---|
| DNA mix from previous step | 20 |
| T7-Bpml-oligo(A)₁₅ primer (10 µM) | 0.5 |
| 10 mM dNTP mix | 0.5 |
| H₂O | 3 |
| Total | 24 |

Incubate at 37 °C for 5 min.
Add 1 µl of Klenow polymerase, mix by tapping
Incubate at 37 °C for additional 55 min.
Inactivate the enzyme at 70 °C for 10 min
Cool to 4 °C
The DNA reaction can be stored at -20 °C for at least 1 year.

### Steps 15-17: In vitro transcription

Set up 50 µl reaction for each DNA sample with RNAmaxx kit as follows:

| **Component** | **Volume (µl)** |
|---|---|
| DNA mix from previous step | 25 |
| 5x Transcription buffer | 10 |
| ATP | 2 |
| CTP | 2 |
| GTP | 2 |
| UTP | 2 |
| 0.75 M DTT | 2 |
| Yeast pyrophosphatase | 0.5 |
| RNAsin | 1 |
| H₂O | 2.5 |
| T7 RNA polymerase | 1 |
| Total | 50 |

Incubate for 16h at 37 °C.
RNA is extracted using the GeneAmp RNA purification kit. RNA was eluted in 22 µl of elution buffer. The final eluate volume is 20 µl.

### Steps 18 - 24: Reverse transcription

Add the following components in a PCR tube

| **Component** | **Volume (µl)** |
|---|---|
| RNA | 20 µl |
| T7-Bpml-oligo(A)₁₅ primer (10 µM) | 2 µl |
| Heat at 65 °C for 10 minute. | |

Plunge the tubes in ice to cool immediately to maintain the linear RNA.
This step denatures the secondary structure of the RNA.

Add the following components for the reverse transcription reaction

| **Component** | **Volume (µl)** |
|---|---|
| RNA | 20 |
| 5xRT buffer | 8 |
| 0.1 M DTT | 4 |
| 10 mM dNTP mix | 2 |
| RNAsin (40U/µl) | 2 |
| Reverse transcripase (200 U/µl) | 2 |
| Total | 40 |

Incubate for 1 h at 42 °C.
Inactivate the enzyme by heating at 75 °C for 10 min.
Cool at 4 °C
The DNA reaction can be stored at -20 °C for at least 1 year.

### Steps 25-28: Second strand synthesis

Add the following components for the second strand synthesis

| **Component** | **Volume (µl)** |
|---|---|
| Mix from the previous step | 40 |
| 10x second strand buffer | 20 |
| 100x BSA | 2 |
| 10 mM dNTP mix | 6 |
| Rnase H (10U/µl) | 1 |
| Taq Polymerase (5U/µl) | 1 |
| Pfu Polymerase (2.5 U/µl) | 0.2 |
| H₂0 | 129.8 |
| Total | 200 |

Incubate in a thermal cycler using the following program

| | |
|---|---|
| 37°C | 5 min |
| 65°C | 1 min |
| 72°C | 30 min |
| Cool to 4°C. | |

Purify the DNA using Qiaspin kit. Elute the DNA in 50 µl volume.
The DNA reaction can be stored at -20 °C for at least 1 year.

### Step 29-33: BpmI digestion

Add the following components to the reaction mix:

| **Component** | **Volume (µl)** |
|---|---|
| DNA from the previous step | 50 |
| NEB reaction Buffer 3 | 6 |
| BpmI (2.5 U/µl) | 2 |
| H₂0 | 2 |
| Total | 60 |

Incubate at 37 °C for 2h.

Heat inactivate the enzyme at 70 °C for 10 min.
Cool to 4 °C.
Purify the digested DNA with Minelute columns. The final eluate volume is 10 µl.

The performance of the different steps can be monitored by using a positive control DNA fragment. Any double stranded DNA fragment with a known sequence of 200-500 bp can be used for this purpose. The addition of a T-tail and the T7 promoter primer can be validated by the change of the molecular mass of the fragment and confirmed by sequencing using internal primers. As a guide Table (i) gives the expected amounts of total amplified RNA and double-stranded DNA for an ERa ChIP from 5,000, 10,000, 100,000 cells and an H3K4me3 ChIP from 1,000 and 10,000 cells. Total RNA was quantified after the in vitro transcription step, and total DNA quantified after the final step just before sequencing. Note that these are independent ChIP experiments and the numbers cannot be extrapolated linearly. However, the InDA data are representative and indicate an apparent experimental amplification factor between about 2 000-fold (for 5 000 cells, ERα) and 400-fold (for 100 000 cells, ERα).

**Table (i) Quantification of yields of RNA and double stranded DNA obtained from representative ChIPs performed with different numbers of cells and two different antibodies.**

| **Number of cells** | **RNA amounts (ng)** | **DNA amounts (ng)** |
|---|---|---|
| *ERa ChIP* | | |
| 100 000 | 103.12 | 54.4 |
| 10 000 | 44.40 | 33.2 |
| 5000 | 24.84 | 14.8 |
| 2 000 000 (no amplification) | | 2.7 |

| *H3K4me3 ChIP* | | |
|---|---|---|
| 10 000 | 49.28 | 27.9 |
| 1 000 | 26.50 | 13.9 |
| 1 000 000 (no amplification) | | 2.3 |
| | | |

The invention will now be described in more detail, with reference to the following figures and experiments.

### DESCRIPTION OF THE FIGURES

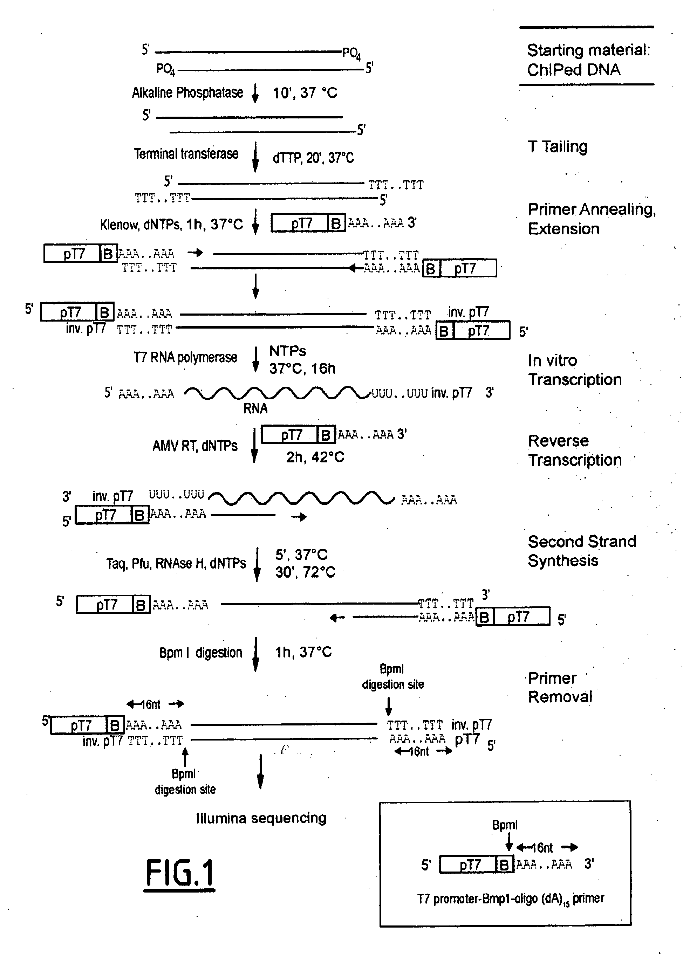

**Figure 1****:** Detailed stepwise description of the LinDA amplification protocol. The 3' ends of the ChIP DNA are dephosphorylated by shrimp alkaline phosphatase for 10 min at 37 °C followed by denaturation of enzyme at 75 °C for 10 min. Subsequently, a limited T tailing of these ends, using dTTP and terminal transferase is performed for 20 min at 37 °C followed by denaturation of the enzyme at 70°C for 10 min. A primer containing the T7 promoter sequence linked to a Bpm1 recognition site ("B" in the illustration) and an oligo (dA)15 tail is allowed to hybridize and the strands are completed by Klenow polymerase at 37 °C for 60 min, followed by denaturation of the enzyme at 70°C for 10 min. The DNA molecules, having T7 promoter attached at both ends, are in vitro transcribed by T7 RNA polymerase for 16h at 37 °C. The RNA produced is purified and subjected to reverse transcription and second strand synthesis by Taq polymerase, RNase H and pfu polymerase mix for 5 min at 37°C and 30 min at 72 °C. The T7-Bpml-oligo(dA) sequence is subsequently cleaved off using Bpml, which cuts 16 nucleotides 3' of its recognition sequence.

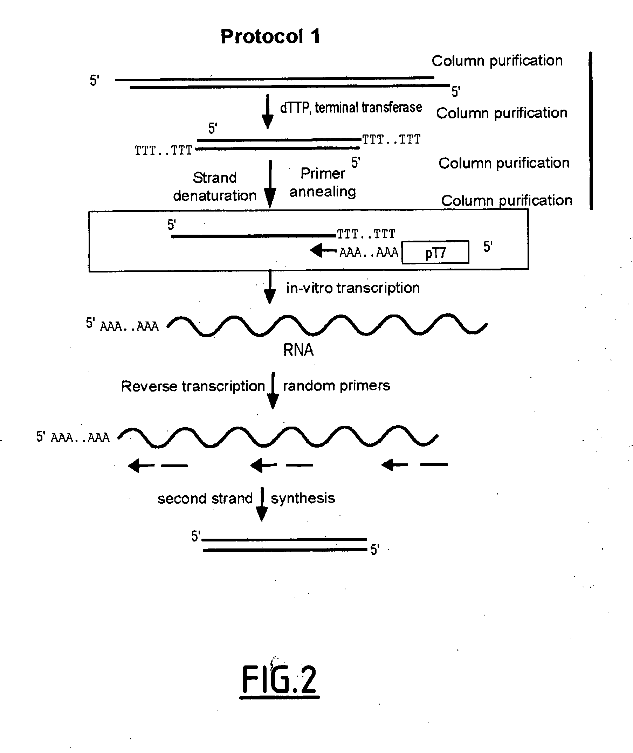

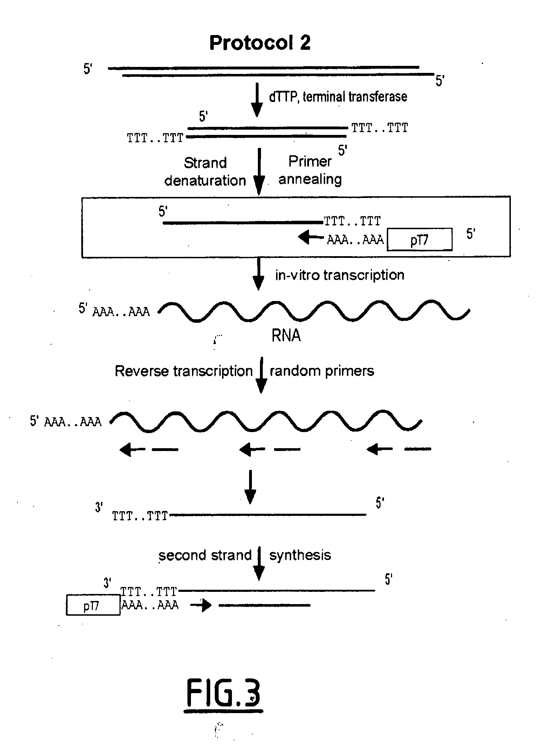

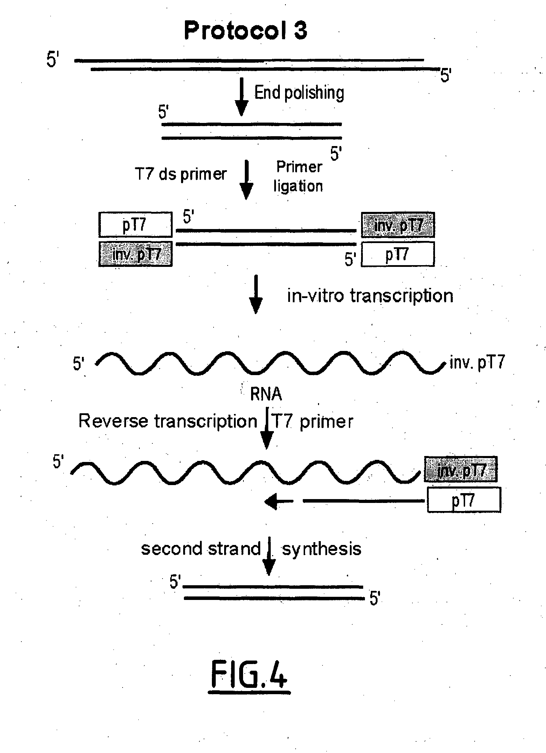

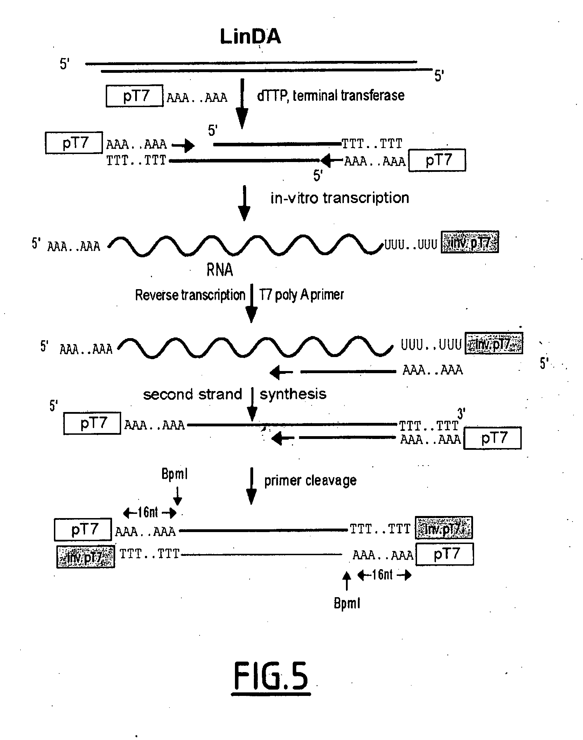

**Figures 2-5****:** Comparison of four T7-based DNA amplification protocols and validation of LinDA. The different protocols display the following features: **Figure 2****:** Prior art method: the classical T7 based protocol described by Liu et al., 2003 and 2008. Note that the DNA is denatured before Klenow polymerase reaction; this results in T7 promoter attachment at only one end, making the reverse transcription complicated and inefficient. A second drawback is that multiple rounds of column purification lead to serious sample loss when the starting material comprises ultra-small amounts DNA. **Figure 3****:** This protocol, developed by the inventors and not previously made public, is a modification of the one described by van Bakel et al., 2008. The inventors introduced a unique buffer, which eliminates the need for multiple rounds of column purification. However, the strand denaturation step prior to Klenow is maintained, thus necessitating random priming for reverse transcription. **Figure 4****:** In this protocol, also developed by the inventors and not known in the prior art, the DNA ends were polished with T7 DNA polymerase and the T7 primer-adapter is ligated to the ends prior to *in vitro* transcription. **Figure 2****:** The LinDA protocol, in which the unique buffer system is combined with the attachment of T7 primer to both ends (no denaturation) thereby increasing efficiency and making reverse transcription with T7 primer possible. For ChIP-seq, a T7 promoter-Bpm1-oligo(dA)15 primer is used to facilitate the removal of T7 and oligo(dA) sequences

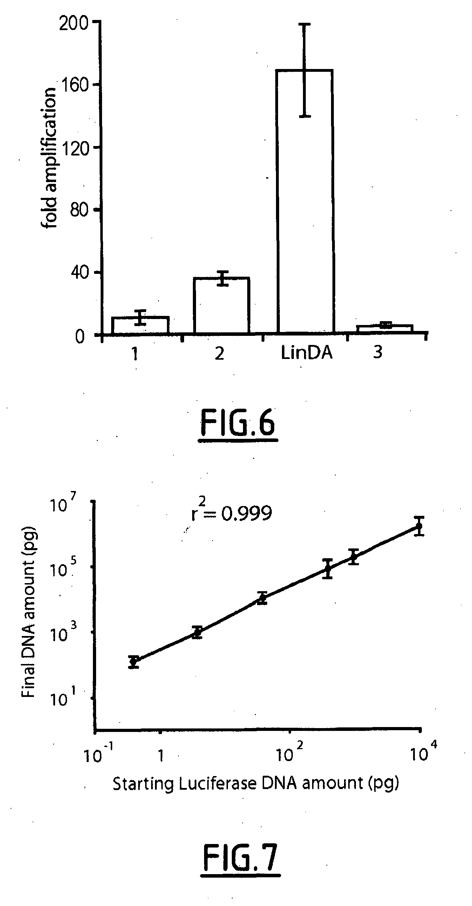

**Figure 6****:** Comparison of the amplification efficiency of the different protocols. LinDA was found to have the optimum combination of high amplication efficiency and ease of operation.
**Figure 7****:** qPCR quantitation of luciferase DNA spiked into salmon sperm DNA sample. Different amounts of a DNA fragment from the luciferase gene (10ng to 0.4pg) were spiked into 100ng of salmon sperm DNA and LinDA was performed.

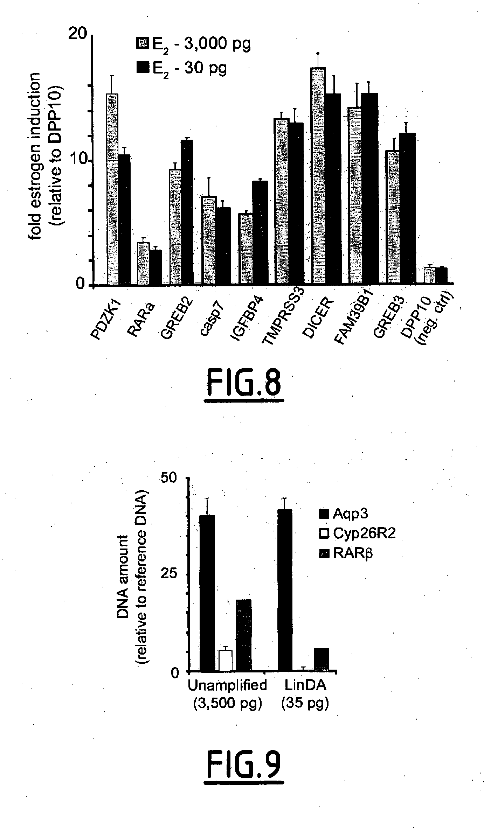

**Figure 8****:** qPCR quantitations of fold increase in ERalpha binding at target genes upon 1 h estrogen treatment. The 3ng unamplified sample was compared with a LinDA amplification of the 30pg sample. Fold occupancies are calculated relative to a "cold" region (*DPP10*).
**Figure 9****:** qPCR validation of RXRalpha targets from LinDA-amplified RXRalpha ChIP samples. Different amounts of RXRalpha ChIPed chromatin from ATRA-treated F9 cells (1 ng, 200pg, 50pg) were amplified with LinDA and the RXR target loci were quantified by qPCR; data are expressed as fold occupancy relative to the *GAPDH* locus.

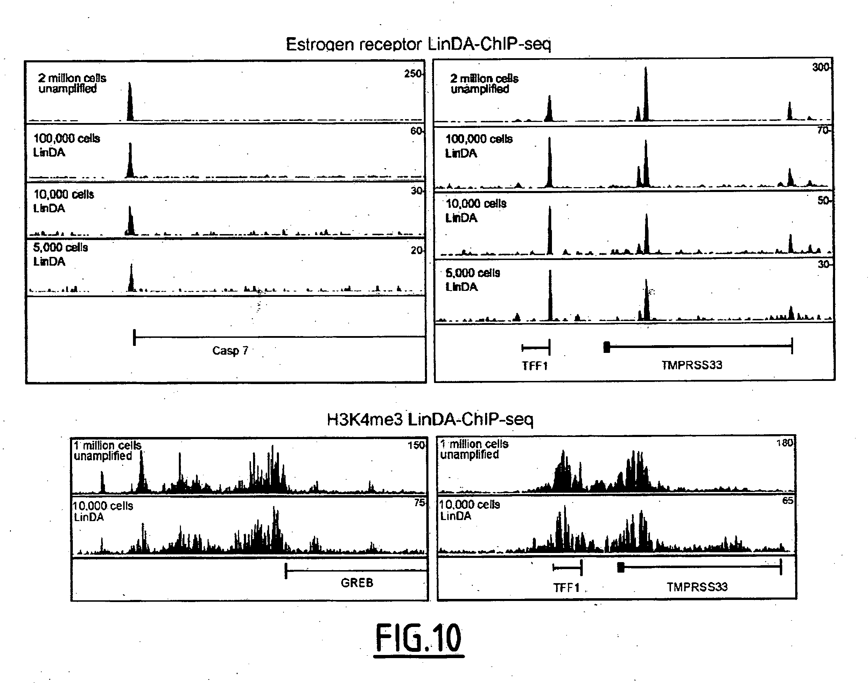

**Figure 10****:** LinDA-ChIP-seq profiling of transcription factor binding and histone modifications from small cell numbers. Top 2 panels: Screenshots illustrating estrogen receptor binding to the casp7 (top left panel) and TFF1 (top right panel) loci from ChIP-seqs. Separate ChIPs were done from 2 million, 100 000, 10 000and 5 000 cells, IPed DNA was separately amplified by LinDA for the 100 000, 10 000 and 5 000 cell samples and sequenced using HiSeq2000 technology. Note the low background. The Pearson correlation coefficients between the corresponding LinDA-amplified samples were r>0.91. Bottom 2 panels: Similar comparison between unamplified ChIPed DNA and LinDA-ChIP-seq for H3K4me3. Screenshots of the GREB and TFF1 loci.

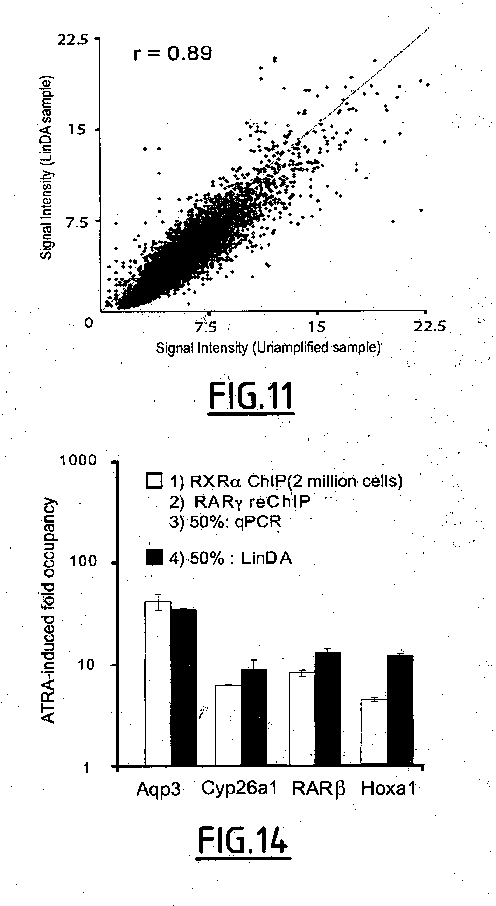

**Figure 11****:** Quantitative comparison of signal intensities obtained from the RXR ChIP-seq of unamplified and LinDA-amplified samples as calculated by seqMINER. The scatter plot shows signals for RXR across 1 kb bins around MACS (p=10⁻⁵) identified peaks. The Pearson correlation coefficient r is indicated.

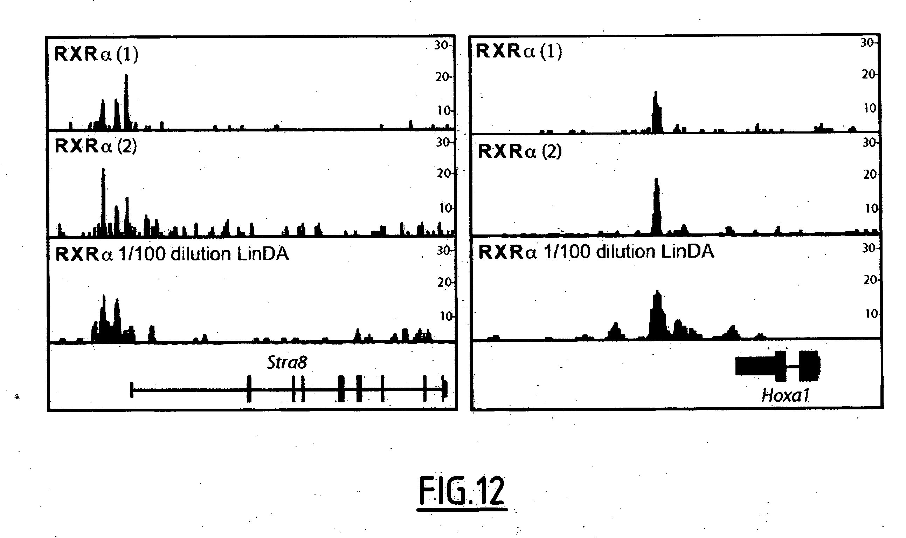

**Figure 12****:** ChIP-seq profiles of the Stra8 and HoxA1 loci are displayed. RXRalpha(1) and RXRalpha(2) are biological replicates. The corresponding LinDA-ChIP-seq profile of a 100-fold diluted RXRalpha(1) sample is shown for comparison.

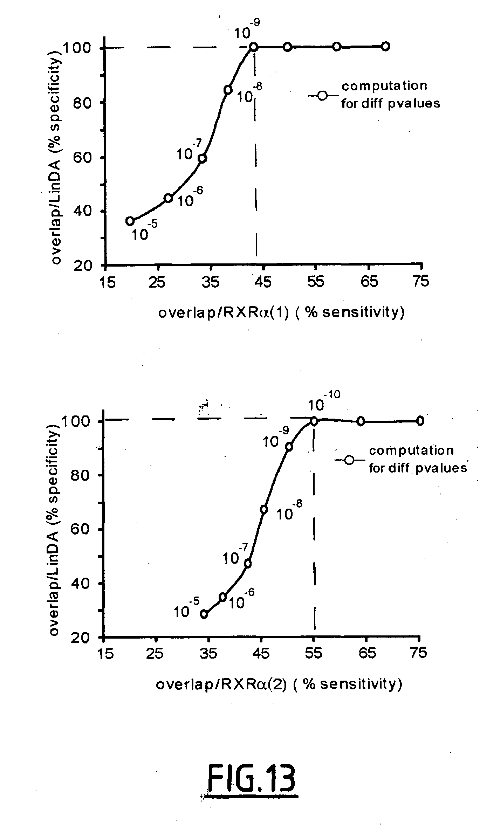

**Figure 13****:** Comparison of Chlp-seq profiling of an RXRaIphaChIP ("RXRalpha(1)"), the corresponding ChIP-seq with 1/100th of RXRalpha(1) ChIP after LinDA amplification, and of a biological repeat ("RXRalpha(2)") Receiver Operating Characteristics (ROC) curves associated to the LinDA amplified sample relative to the RXRalpha1) (top panel) and RXRalpha2) samples (bottom panel).
**Figure 14****:** ReChIP of RARgamma/RXR heterodimer using LinDA. qPCR validation of standard RXR targets from LinDA amplified reChIP (RXRalpha, RARgamma) samples as compared to the unamplified sample. Error bars are derived from technical replicates.

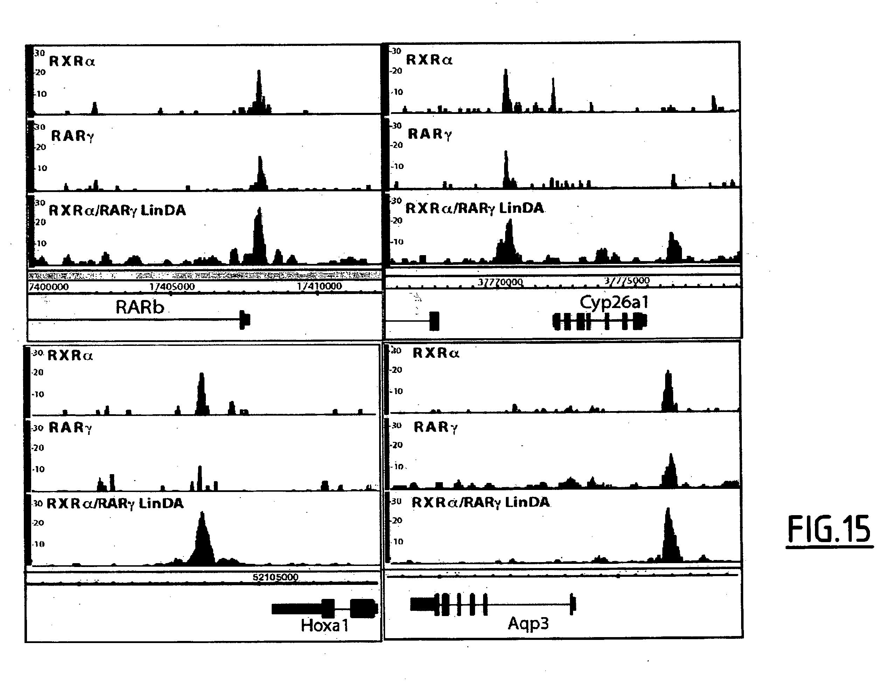

**Figure 15****:** Genomic display of the ChIP-seq data obtained from RXRalpha, RARgamma and LinDA-amplified reChIP samples. ChIP-seq profiles around the *RARb*, *Cyp26a1, Hoxa1* and *Aqp3* genes reveal conservation of the profile.

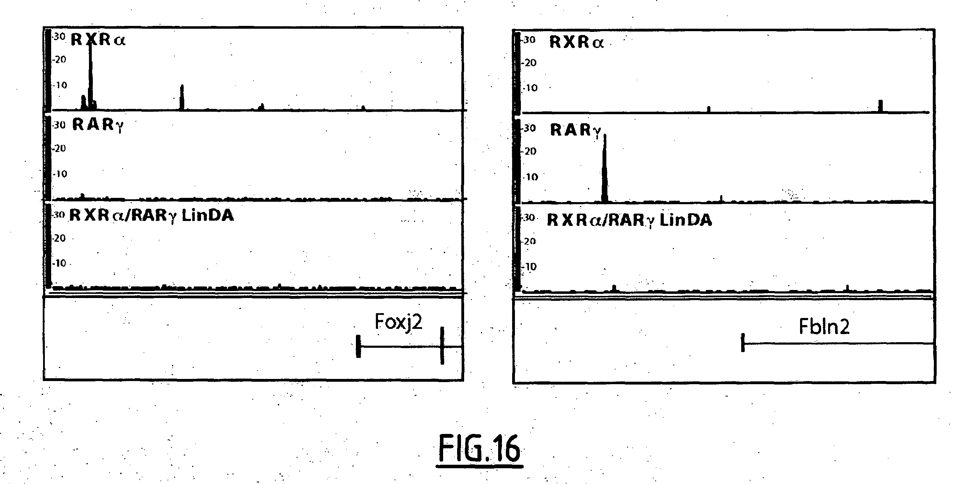

**Figure 16****:** Exclusive binding of RXRalpha (bottom left) or RARgamma (bottom right) most probably due to the binding of heterodimers with partners other than RARgamma and RXRalpha, respectively. Note that, as expected, no RXRalpha - RARgamma heterodimer is seen at these loci in the LinDA-reChIP-seq profile.

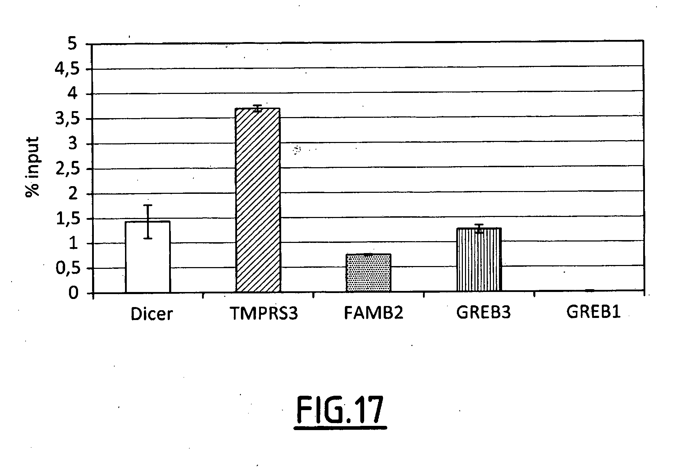

**Figure 17****:** Validation of PAT-ChIP-LinDA. The graph shows H3 acetylation of % of input. A single 5 µm FFPE section from a tumor that originated upon xenografting of human MCF-7 breast cancer cells onto immunoincompetent mice was cut with a microtome and directly collected in a 1.5 ml sterile tube. PAT-ChIP-LinDA was performed with an antibody that detects acetylated histone H3 (pan-H3ac) as described above to identify chromatin domains that harbor acetylated histone H3. The obtained ChIP'ed and LinDA amplified DNA was subjected to real time quantitative PCR with 5 different primer pairs corresponding to estrogen-receptor target genes for which the H3 acetylation status is known. The first four primers (Dicer, TMPRSS3, FAMB2, GREB3) define loci at transcription start sites (TSSs) or the gene body, while the GREB1 locus is located 30 kb upstream of its cognate TSS. Amplification - which reveals the presence of acetylated H3 at these loci in the tumors - was observed only with LinDA-amplified material; non-amplified or 'no antibody' samples did not show any DNA.

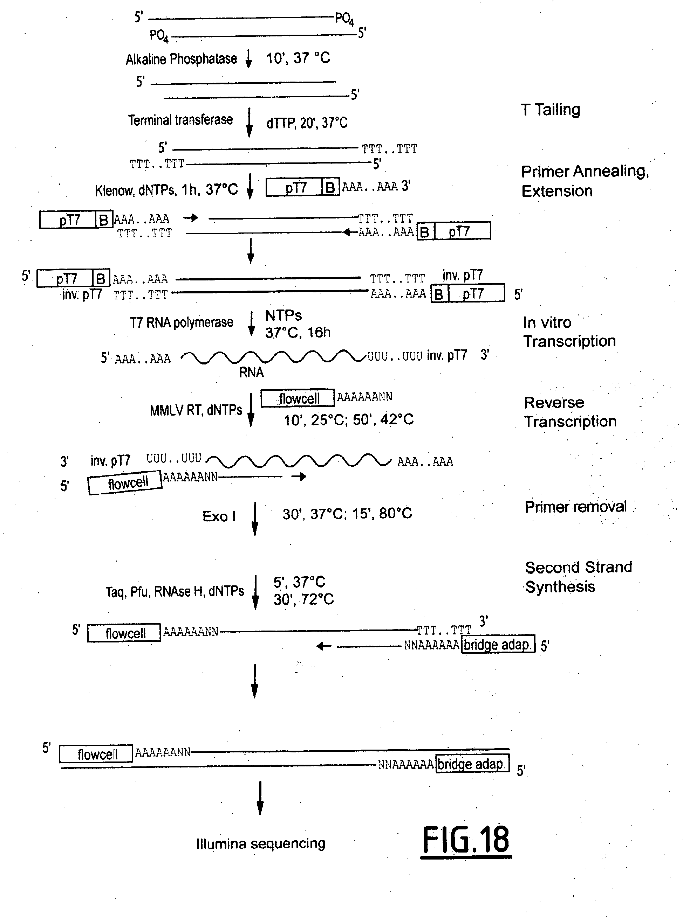

**Figure 18****:** Detailed stepwise description of an embodiment of the LinDA amplification protocol, ChIP-LinDA-seq, in which the flowcell and bridge adapter primers used in the Illumina sequencing technology are incorporated into the procedure, enabling the direct production of a DNA library with the adapters attached, ready for sequencing.

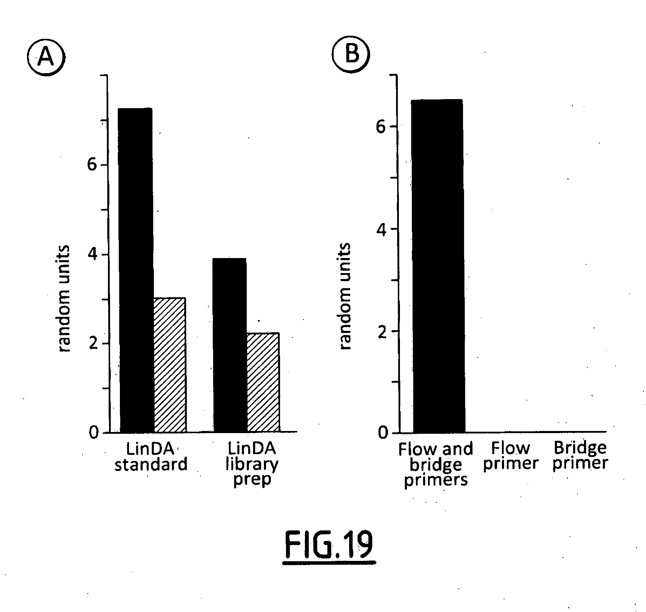

**Figure 19****:** Validation of ChIP-LinDA-seq in which the sequencing library is integral part of the procedure. ChIP assays were performed with mouse F9 teratocarcinoma cells using anti-RXR antibodies and LinDA was performed according to the invention. Real time qPCR assays demonstrate the efficiency of the library preparation. (A): Comaprison between standard LinDA and the "new" LinDA library preparation for 2 standard RXR target genes (Aqp3 (black) and RARb (hatched). Similar amounts of DNA are recovered at the end of either procedure. (B): Efficiency of the inclusion of Illumina adapters. Only the double adapter attached molecules are observed in the final DNA while singly labeled DNA is not seen.

### EXPERIMENTAL

### Methods

### Cell culture

F9 EC cells were cultured in DMEM supplemented with 10% FCS and 40µg/ml gentamicin. Cells were seeded in gelatin-coated tissue culture plates (0.1%) and *all*-trans retinoic acid (ATRA) was added to a final concentration of 1 µM.

Human H3396 cells were grown in RPMI (with 25mM HEPES) supplemented with 10% fetal calf serum and gentamicin. For induction, cells were maintained in estrogen (E₂)-deficient conditions (charcoal-stripped serum, no phenol red) for 72h; induction was with 10nM E₂ for 1h.

### Chromatin immunoprecipitation (ChIP)

Cells were fixed with 1% para-formaldehyde (Electron Microscopy Sciences) for 30 min at room temperature. ChIPs were performed following standard conditions: Chromatin sonication (200-500bp length) and IP in lysis buffer (50mM Tris-Cl pH=8, 140mM NaCl, 1mM EDTA, 1% Triton, 0.1% Na-deoxycholate) complemented with protease inhibitor cocktail (Roche 11873580001); 2xwashes with lysis buffer; 2xwashes with lysis buffer containing 360mM NaCl; 2xwashes with washing buffer (10mM Tris-Cl pH=8, 250mM LiCl, 0.5%NP-40, 1 mM EDTA, 0.5% Na-deoxycholate ); 2xwashes with 1xTE; elution at 65°C; 15 minutes in elution buffer (50mM Tris-Cl pH=8, 10mM EDTA, 1% SDS). RXRalpha and RARgamma were IPed with in house validated monospecific polyclonal antibodies directed against synthetic peptides (mRXRalpha: PB105 , mRARgamma: PB288). ERalpha and H3K4me3 IPs were done with anti-ERalpha (sc-543; Santa Cruz) and AB-8580 (Abcam), respectively. The small cell number ChIPs were performed as per the original protocol described above except for the antibody amounts (anti-ERalpha: 2µg for 2 M and 100k cells, 0.5 µg for 10k and 5k cells; anti-H3K4me3: 1 µg for 1 M cells, 0.25 µg for 10k cells). IP-enrichment of chromatin fragments was defined relative to the input control and/or relative to a "cold" reference region; the corresponding data are expressed as "fold occupancy (FO)" using quantitative real time PCR (qPCR, Roche LC480; Quantitect, Qiagen).

### ChIP from paraffin sections

### Deparaffination:

Single 5 µm FFPE section from a MCF-7 xenografted tumor was cut with a microtome and directly collected in a 1.5 ml sterile tube.
1 ml of Histolemon (CARLO ERBA REACTIFS) was added and the tube was rocked from side to side for 5 min at RT (room temperature).
The tube was centrifuged at 12,000xg for 10 minutes and the supernatant was discarded. 1 ml of Histolemon (CARLO ERBA REACTIFS) was added and the tube was rocked from side to side for 5 min at RT (room temperature).
The tube was centrifuged at 12,000xg for 10 minutes and the supernatant was discarded. 1 ml of 95% ethanol was added and the tube was rocked from side to side for 5 min at RT (room temperature).
The tube was centrifuged at 12,000xg for 10 minutes and the supernatant was discarded.
1 ml of 70% ethanol was added and the tube was rocked from side to side for 5 min at RT (room temperature).
The tube was centrifuged at 12,000xg for 10 minutes and the supernatant was discarded. 1 ml of 50% ethanol was added and the tube was rocked from side to side for 5 min at RT (room temperature).
The tube was centrifuged at 12,000xg for 10 minutes and the supernatant was discarded. 1 ml of 20% ethanol was added and the tube was rocked from side to side for 5 min at RT (room temperature).
The tube was centrifuged at 12,000xg for 10 minutes and the supernatant was discarded. 1 ml of water was added and the tube was rocked from side to side for 5 min at RT (room temperature).
The tube was centrifuged at 12,000xg for 10 minutes and the supernatant was discarded. 1 ml of water was added and the tube was rocked from side to side for 5 min at RT (room temperature).
The tube was centrifuged at 12,000xg for 10 minutes and the supernatant was discarded. 400 µl of SDS lysis buffer (1% SDS, 50 mM Tris pH 8, 10 mM EDTA) was added to the tissue pellet and ipetted up and down.
The mixture was sonicated in an ice bath until fragments of around 500 bp were obtained. CHIP was performed under standard conditions with 0.25 µg of antibody recognizing acetylated histone H3 ('pan-H3ac') overnight at 4 °C.
DNA recovered after decrosslinking and purification was dissolved in 14 µl of water and directly used for linear DNA amplification (LinDA).
CHIP and amplification steps were confirmed with qPCR.

### Sequential reChIP assay

For reChIPs, the first antibody (anti-RXRalpha) was covalently linked to the sepharose protein A (Sigma P92424) using disuccinimidyl suberate (DSS). The covalently linked Ab-beads were washed with ethanolamine (0.1 M), followed by glycin at pH 2.8. Beads pre-washed with 50mM sodium borate at pH 8.2 and PBS were incubated overnight at 4°C as for regular ChIPs. Following standard washing, elution was performed with 10mM DTT (30 min, 37°C). Eluates from at least 4 ChIPs were combined, diluted at least 30 times with lysis buffer (containing protease inhibitors), and incubated overnight with the second antibody (anti-RARgamma) and protein A beads at 4°C. The subsequent steps were performed as for regular ChIPs.

### LinDA protocol

DNA obtained from ChIP was first dephosphorylated using 1 U shrimp alkaline phosphatase (Promega) for 20 min at 37°C. The unique buffer used in the protocol was 20mM Tris-acetate, 10mM magnesium acetate, 50mM potassium acetate, 1mM dithiothreitol (pH 7.9). The enzyme was inactivated by heating at 70°C for 10 min. DNA was then T-tailed by addition of 5µM T tailing mix (dTTP and ddCTP); 20U (20 micromoles) terminal transferase (NEB) and 5mM CoCl₂ at 37°C for 20 min. The enzyme was once again heat inactivated at 70°C for 10 min and 5 pmole T7 promoter-Bpml-oligo(dA)₁₅ primer was added to the mix and allowed to anneal at 37°C for 5 min. Extension and completion of the double strand was performed by the addition of 10U Klenow polymerase (NEB) and 0.2mM dNTPs for 1 h. After heat inactivation of the enzyme, the components of the RNAmax™ *in vitro* transcription mix (Stratagene) (1x proprietary RNAmax™ transcription buffer, 4 mM of each rUTP, rGTP, rATP, rCTP;0.03M DTT, 0.5 µl 0.75 U/µl yeast inorganic pyrophosphatase, 1 µl RNaseblock, 1 µl of 200U/µl T7 RNA polymerase) were added and the reaction was performed overnight at 37°C. RNA was extracted with the Sigma RNA extraction kit and eluted in a volume of 20µl. Reverse transcription was performed using the same T7 promoter-Bpml-oligo(dA)₁₅ primer in a buffer comprising 50 mM Tris HCl (pH 8.3), 75 mM KCl, 3 mM MgCl2, 20 mM DTT using the same T7 promoter-Bpml-oligo(dA)₁₅ primer and Superscript kit (Invitrogen) at 42°C for 2h. Second strand synthesis was performed in a buffer comprising 20 mM Tris HCl (pH 8.8), 10 mM (NH4)SO4, 10 mM KCl, 2 mM MgSO4, 0.1% Triton X100, 0.1 mg/ml BSA using 5U RNAse H (NEB), 5U Taq polymerase (Roche) and 0.25U Pfu polymerase (Stratagene) at 37°C for 5 min followed by 72°C for 30 min. DNA was purified using QiaSpin columns (Qiagen). T7 primed ends were excised by digesting the DNA with 10U Bpml which cuts 16 nucleotides 3' of its recognition sites and removes sequences introduced by the initial T tailing. Samples were then directly processed for Illumina sequencing.

### Illumina sequencing and data processing

Library preparation and the Illumina sequencing was performed according to the manufacturers protocol. RXRalpha/RARgamma sequencing was done with G2AX; ERalpha and H3K4me3 assays were sequenced on the HiSeq2000. Sequenced reads were mapped to the mm9 mouse genome or to the hg19 human genome assembly for RXRalpha/RARgamma and ERalpha/H3K4me3 profiling, respectively. Peaks were identified using MACS¹⁴ (http://liulab.dfci.harvard.edu/MACS/). Signal intensity correlations were performed using seqMINER¹⁵. GC content comparison of unamplified and LinDA-amplified ChIP-seq profiles was done by a genome-wide sliding window analysis to compute tag count intensities and %GC content in 1 kb bins.

### Results

### Validation of LinDA efficacy

To validate LinDA a 404 bp DNA fragment of the luciferase gene (Luc) was amplified. LinDA generated the predicted 527 bp fragment, and sequencing confirmed the presence of the T7 promoter at both extremities. To quantify LinDA under conditions where target DNA is "contaminated" by a large excess of heterologous genomic DNA increasing amounts of Luc DNA (0.4pg to 10ng) were co-amplified in the context of 100ng sheared salmon sperm DNA. Quantitative PCR analysis revealed a highly reliable ~300-fold amplification over 3 logs of sentinel DNA concentrations (Fig. 7).

That LinDA reliably amplifies ChIPed DNA was confirmed by comparing estrogen-induced target gene binding of estrogen receptor-alpha (ERalpha) by quantitative PCR to 9 different target loci, which were identified in a separate ChIP-seq study using H3396 human breast cancer cells. Indeed, the fold induction of ERalpha occupancy (relative to the "silent" locus *DPP10*) at these sites was virtually indistinguishable when 3ng of the ChIPed DNA were compared with a 30pg aliquot amplified by LinDA (Fig. 8). Similar results were obtained for binding sites of retinoid X receptor-alpha (RXRalpha) heterodimers ChIPed with anti-RXRalpha antibodies after exposure of F9 mouse teratocarcinoma cells to *all-trans* retinoic acid (ATRA). Quantitative PCR for nine binding sites revealed very similar fold occupancies of RXRalpha (heterodimers) relative to *GAPDH* when the LinDA amplifications from 1ng, 200pg and 50pg of ChIPed DNA were compared (Fig. 9), thus revealing a very similar rate of amplification.

LinDA can be efficiently used with the standard ChIP protocol if antibody amounts are adjusted. Indeed, ChIP-seq of ERalpha can be performed with as few as 5,000 cells, identifying about 70% of the high confidence peaks, and global profiling of H3K4me3 has been done with 10,000 cells (Fig. 10; Table 2). Modifications of the LinDA-ChIP-seq protocol and increasing sequencing depths are likely to reduce the numbers required for global profiling below 1,000 cells. It is important to point out that in contrast to PCR-based amplification techniques, LinDA shows no GC-amplification bias.

Profiles generated by Illumina sequencing were then compared from (i) 3.5ng of a RXRalpha-specific ChIP from F9 cells ["RXRalpha(1)"], (ii) a biological replicate done at a different time ["RXRalpha(2)"], and (iii) the LinDA-generated library from 35pg of RXRalpha(1) (Table 1). Statistically significant binding sites in RXRalpha(1) and the corresponding LinDA ChIP-seq were annotated by using MACS¹⁴ using 10⁻⁵ as p-value cutoff. To evaluate the similarity between samples, MACS-annotated regions were compared similarly as described¹¹ in the context of their read-count intensities in 1 kb windows surrounding MACS-annotated peaks using seqMINER¹⁵, revealing a Pearson correlation coefficient of 0.89 (Fig. 11). Visual comparison of the ChIP-seq profiles showed indeed excellent concordance (Fig. 12). While this correlation was comforting, the binning approach may be sub-optimal for TFs, which bind to short well-defined sequences, as the resulting cohort may be dominated by peaks with low tag counts and/or low p-values. We therefore established ROC curves by defining the overlaps between MACS predicted peaks at different p-values. The goal was to define the p-value at which 100% of the LinDA peaks were equally detected in ChIP-seqs of non-amplified DNA. This analysis showed that 100% specificity, i.e. all LinDA peaks are detected in the ChIP-seq of the original sample, is attained at p-value of 10⁻⁹ for RXRalpha(1) and 10⁻¹⁰ for the independent biological replicate RXRalpha(2). At these p-values about half of the RXRalpha peaks are detected by LinDA (Fig. 13). Clustering of the top 200 sites revealed that LinDA ChIP-seq corresponds to a biological replicate and is detected with highly similar efficiency sites annotated to harbour RXR family response element.

Together these data show that LinDA permits 100% reliable retrieval of genome-wide TF binding sites from pg amounts of ChIPed DNA. Using longer reads and increasing the number of (mappable) reads, the sensitivity of LinDA is likely to increase further.

### Use of LinDA in re-CHIP analysis

While ChIP-seq analyses directly reveal global TF binding patterns, these factors frequently act in concert with others. Often TFs function are heterodimers, like the RXR family or they are members of high-molecular-weight complexes, or they bind to targets cooperatively with other factors. The analysis of co-binding may therefore be of importance to reveal sub-programs linked to a particular TF complex/modification. One possibility to study cooperative chromatin binding genome-wide is the use of re-ChIP, which involves a second IP performed on the first ChIP sample with a different antibody. However, using current technologies re-ChIPs yield very small amounts of DNA and the first ChIP has to be done with a huge amount of cells, which is costly and time-consuming, if possible at all.

To assess the utility of LinDA for re-ChIPs the inventors set out to define the binding site repertoire of the RXRalpha-RARgamma heterodimer relative to the global binding patterns of RXRalpha and RARgamma in F9 cells 2h after ATRA-induced differentiation. RXRalpha ChIPed chromatin was re-ChIPed with antibodies specific for RARgamma. As the IPed DNA could not be quantified by Qubit (detection limit 100pg), half of it was subjected to LinDA yielding ~30ng DNA. Using one half of the non-amplified re-ChIP the ATRA-induced binding to 4 known ATRA-responsive loci was compared with the LinDA-amplified re-ChIP, revealing comparable induction of the presumptive RXRalpha-RARgamma heterodimer (Fig. 14). Notably, sequencing of the LinDA-amplified re-ChIP (Table 1) yielded 3683 MACS-predicted peaks (p-value 10⁻⁵) of which 2277 overlapped with the cohorts of peaks obtained by separate ChIP-seq analysis of RXRalpha and RARalpha. Visual inspection of re-ChIP LinDA profiles with the separate ChIP-seqs of RXRalpha and RARgamma showed high concordance (Fig 15). Sites that are outside of the 2277 cohort most likely correspond to binding of RXRalpha heterodimers with RARalpha or RARbeta, and of RARgamma heterodimers with RXRbeta or RXRgamma. Indeed, such sites are not retrieved by the LinDA re-ChIP-seq (Fig. 16), thus revealing the heterodimer selectivity of the approach.

**Table 1: ChIP-seq and LinDA-ChIP-seq sequence data. Experiments performed using the G2AX platform.**

| **ChIP-seq samples** | **Reads (36bp)** | **Mappable Reads** | **Uniquely Aligned** |
|---|---|---|---|
| RXRalpha(1) | 27,647,520 | 13,941,574 | 12,977,749 |
| LinDA with 1/100 of RXRalpha (1) | 24,634,560 | 8,481,378 | 7,211,018 |

| **Re-ChIP-seq & LinDA**-**ChIP**-**seq** | | | |
|---|---|---|---|
| RXRalpha(2) | 13,619,647 | 6,520,940 | 4,856,220 |
| RARgamma | 15,545,924 | 6,125,803 | 5,257,056 |
| RXRalpha/RARgamma reChIP - LinDA | 23,561,400 | 8,010,990 | 6,747,941 |

**Table 2: ChIP-seq and LinDA-ChIP-seq sequence data. Experiments performed using the HiSeq200 platform.**

| **ChIP-seq samples** | **Reads (50bp)** | **Mappable Reads** | **Uniquely Aligned** |
|---|---|---|---|
| ERalpha (2M cells) | 31,649,051 | 27,370,644 | 24,507,052 |
| H3K4me3 (1M cells) | 30,851,932 | 27,913,395 | 24,800,042 |

| **LinDA-ChIP-seq** | | | |
|---|---|---|---|
| ERalpha (100k cells) | 26,091,833 | 20,346,928 | 16,044,435 |
| ERalpha (10k cells) | 26,172,339 | 20,031,608 | 15,801,230 |
| ERalpha (5k cells) | 23,108,536 | 16,390,466 | 12,900,877 |
| H3K4me3 (10k cells) | 26,534,833 | 22,395,440 | 18,466,449 |

### Comparison of LINDA with alternative protocols

The inventors compared three modifications of the original T7 linear amplification protocol of Liu et al. (Fig. 2-4) with LinDA (Fig 5). The highest amplification was obtained by LinDA (Fig. 6), which combines several modifications of the other protocols, as follows:
(i) Introduction of an inverse T7 primer to the 5' end of the RNA due to suppression of thermal denaturation of DNA prior to the first DNA polymerase reaction. The subsequent use of the T7 promoter-oligo(dA) primer-adapter for double-strand synthesis regenerates a DNA with T7 primers at both ends that is ready for the next round of amplification.
(ii) Buffer and enzyme concentrations have also been optimised, allowing for the use of a single buffer for multiple steps of the procedure, which obviates the need for column purification.
(iii) In the experiments carried out by the inventors, in order to optimize the amplified DNA for sequencing, a T7 promoter-Bpml-oligo(dA) primer adapter was used, which facilitates removal of the T7 promoter and 16 additional nucleotides of the oligo(dA) stretch.
(iv)

In the classical T7 based protocol described by Liu et al., 2003 and 2008 (see Fig 2) and the method of Bakel et al, 2008, the DNA is denatured before the Klenow polymerase reaction, which produces DNA fragments with a T7 promoter sequence at only one end of the fragment (not at both ends, as in the method of the present invention). This results in T7 promoter attachment at only one end of the DNA, making the reverse transcription complicated and inefficient. A second drawback is that multiple rounds of column purification lead to serious sample loss when the starting material comprises ultra-small amounts DNA. The superiority of the present method can be clearly seen from Fig 6, which demonstrates that LinDA produced hugely greater fold amplification than the method of Liu et al (column 1) or even an improved version of the method of Bakel et al (column 2).

### PAT-ChIP-LinDA-sea procedure for genome-wide analyses from paraffin-embedded tissue sections

Use of formalin-fixed paraffin embedded samples (FFPR) for chromatin immunoprecipitation (termed PAT-ChIP) and PAT-ChIP-seq is described in Ceschin et al (2011), Genes Dev 25, 1132-1146. In this PAT-ChIP-seq procedure, multiple 10 µm sections (4 to 6) were combined to perform a single ChIP with a histone antibody and the sample was sequenced after multiple rounds of amplification by PCR (up to 20 cycles). Here, LinDA (linear amplification) has been combined with PAT-ChIP to establish a PAT-ChIP-LinDA-seq procedure, which greatly increased the sensitivity of the assay, decreased the amount of starting material and removed all PCR amplification steps that may lead to sequence bias. With this technology all ChIP and ChIP-seq studies, in particular epigenome analyses and genome-wide mapping of transcription factor binding sites, can be performed with a single 5 µm tissue section; this technology can be extended to ChIP and ChIP-seq from laser-dissected areas or cells of tissue sections. The PAT-ChIP-LinDA technology has been validated by defining histone H3 acetylation in a single 5 µm tissue FFPE section derived from a human breast cancer cell (MCF7) xenograft (Fig. 17).

### ChIP-LinDA-seg with integration of the sequencing library preparation

PAT chip can be integrated to the LinDA procdure and the library preparation in such a way it is possible to start from a tissue section and end up with a library that can be used directly for Illumina sequencing without the need of separate library preparation in consecutive streamlined steps.

### LinDA library prep

Library preparation for Illumina technology-based sequencing, and other formats like Roche 454 or SoLid, utilize the ligation of special adapter fragments to the DNA followed by multiple rounds of PCR amplifications to generate a doubly tagged DNA library. The inventors have included the special adapter primers into the LinDA procedure to obtain a DNA library with the adapters attached. No PCR amplification is involved, thus avoiding any PCR bias. As this sequencing library preparation is entirely integrated to the LinDA procedure, it will greatly reduce time and costs, and improve efficiency and fidelity of the sequencing reaction. Validation of the procedure is shown in Fig. 18; a flow scheme of the ChIP-LinDA-seq procedure is shown below.

RNA from the standard LinDA in vitro transcription procedure is reverse transcribed using a special primer which contains the Illumina flowcell complementary sequences followed by 9 'A's. After the RT step the primer is removed by Exonuclease 1 digestion. Second strand synthesis is performed using the second special adapter primer. This results in double stranded molecules containing unique adapter primers on either ends and therefore are ready for sequencing.

### Reverse transcription

| **Component** | **Volume (µl)** | |
|---|---|---|
| RNA | 10 µl | |
| FLOWCELL primer (10 µM) | 1 µl | |
| Heat at 65 °C for 10 minute, Plunge the tubes in ice to cool immediately | | |

| **Component** | **Volume (µl)** | |
|---|---|---|
| RNA | 11 | 22 |
| 5xRT buffer | 4 | 8 |
| 0.1 M DTT | 2 | 4 |
| 10 mM dNTP mix | 1.5 | 2 |
| RNAsin (40U/µl) | 0.5 | 2 |
| Reverse transcripase (200 U/µl) | 1 | 2 |
| Total | 20 | 40 |

Incubate for 10 min at 25 °C, 50 min at 42 °C, 75 °C for 10 min, Cool to 4 °C

| **Primer removal** | | |
|---|---|---|
| **Component** | **Volume (µl)** | |
| Mix from the previous step | 20 | 40 |
| 10x Thermopol buffer | 5 | 5 |
| Exonuclease I | 1 | 1 |
| Rnase H | 1 | 1 |
| H₂O | 23 | 3 |
| Total | 50 | 50 |

Incubate for 30 min at 37 °C, 15 min at'80 °C, Cool to 4 °C

| **Second strand synthesis** | |
|---|---|
| **Component** | **Volume (µl)** |
| Mix from the previous step | 50 |
| BRIDGE primer (10 µM) | 1 |
| 10x Thermopol buffer | 5 |
| 100x BSA | 1 |
| 10 mM dNTP mix | 3 |
| Taq Polymerase (5U/µl) | 0.5 |
| Pfu Polymerase (2.5 U/µl) | 0.5 |
| H₂O | 39 |
| Total | 100 |

Incubate in a thermal cycler using the following program

| | |
|---|---|
| 25 °C | 5 min |
| 37 °C | 5 min |
| 72°C | 30 min |

Cool to 4 °C, Purify the DNA using Qiaspin kit. Elute the DNA in 50 µl volume.

### Linear DNA amplification applied to Chromatin Interaction Analysis by Paired-end Tag sequencing : LinDA-ChIA-PET-seq

Revealing the three-dimensional chromatin organization at a high resolution is currently possible thanks to the combination of proximity-mediated ligation methods with massive parallel sequencing. One of the most recent variants, able to assess a global view of the chromatin organization under an immunoprecipitated target context is described in Fullwood (2009), Nature 462, 58-64. This methodology, named "Chromatin Interaction Analysis by Paired-end Tag sequencing" or ChIA-PET follows and standard chromatin immunoprecipitation approach, thus instead of the elution step, the immunoprecipitated chromatin is split in two and ligated to specific linkers providing a unique barcode information. Then, split samples are collected again in a single vial where a proximity-mediated ligation process is induced under diluted conditions. The circularized events retrieved after this process, are then linearized by using a restriction site located in the previously introduced linkers. Importantly, the restriction enzyme in use (i.e. Mmel) cleaves the DNA in an upstream manner, thus releasing in this manner, linker molecules attached to genomic sequences (20 nt length in the case of Mmel). Finally, a second chromatin immunoprecipitation step is performed, this time targeting the incorporated linkers, which contain a biotin molecule. The captured DNA fragments are then capped by sequencing adapters by following a ligation-mediated approach, then amplified by following a 25 cycles PCR.

In this context, the inventors have modified the current ChIA-PET procedure by incorporating two major steps:
(i) the incorporation of in vitro transcription by following the LinDA procedure
(ii) the incorporation of the required adapters for sequencing (flow cell hybridization adapter and bridge amplification adapter) by following the above described LinDA library preparation procedure.

These modifications were validated in an assay in which a ChIA-PET library has been generated and where the presence of the flow cell hybridization and bridge amplification adapters were confirmed by a quantitative PCR approach.

### Development of LinDA-based technologies for the study of nuclear architecture

While ChIA-PET assays have been shown to be powerful for assessing the long distal chromatin interactions in a high-resolution manner; a certain number of technical aspects leave space for improvement of previously used protocols. In fact, as a consequence of its design (two subsequent chromatin immunoprecipitation steps), more than 70 million cells are required per traditional ChIA-PET assay, thus becoming a limiting factor when trying to address the chromatin architecture in cells other than those generated from *in vitro* cultured model systems. Furthermore, aside from the large amount of cells required, the traditional procedure still requires major PCR-based DNA amplification prior to massive parallel sequencing.

For these reasons, the implementation of the LinDA linear DNA amplification to allow high-fidelity ChIA-PET profiling with low amounts of initial material will allow analysing the chromatin architecture in conditions in which the starting material (cells, tissue, etc.) becomes a limiting factor. Importantly, this method, based on a T7 RNA polymerase-based amplification approach, is devoid of GC bias, in contrast to PCR-based techniques known to suffer from biased amplification of GC-rich sequences.

In conclusion, LinDA is a simple HTS-compatible method suitable for the amplification of ultra-small DNA quantities, which does not introduce artefacts or bias. Currently LinDA-ChIP-seq profiling of TFs and histone modifications have been done with a few thousand cells using the standard ChIP protocol; improvements of the ChIP procedure, increasing sequencing depth and adding further round(s) of LinDA are likely to permit such assays for (a few) hundred cells to reveal the robust binding loci. LinDA will also facilitate chromatin conformation capture-based technologies for the mapping of long range interaction. While LinDA can be applied to amplification of any source of DNA, it will be particularly useful to analyze TF complexes, histone modification and chromatin remodelling in very small organismal compartments, such as stem and cancer-initiating cells.

## Claims

1. A method of linear DNA amplification comprising the steps:
(i) T-tailing DNA ends of double-stranded DNA fragments in a sample;
(ii) annealing to said fragments primers comprising an RNA polymerase promoter site upstream of a poly-A tail, wherein said primers anneal to the poly-T ends of said fragments;
(iii) using a 5'-3' DNA polymerase to synthesise DNA complementary to the primer overhangs, to create double-stranded DNA fragments with an RNA polymerase promoter site at both ends;
(iv) in vitro transcribing said DNA using an RNA polymerase which binds to said RNA polymerase promoter site;
(v) reverse transcribing the RNA products of step (iv) to create single-stranded DNA products;
(vi) creating double stranded DNA fragments by second strand synthesis of the single-stranded DNA of step (v);
(vii) optionally, repeating steps (iv)-(vi).

2. A method according to claim 1 comprising the steps:
(i) incubating the double-stranded DNA sample with alkaline phosphatase in order to dephosphorylate 3' ends;
(ii) inactivation of alkaline phosphatase by heat treatment of the sample;
(iii) adding to the sample a terminal transferase and dTTPs and incubating for T-tailing of DNA ends;
(iv) inactivation of terminal transferase by heat treatment of the sample;
(v) adding to the sample primers comprising an RNA polymerase promoter site upstream of a poly-A tail and incubating to allow annealing of the primers to the sample DNA;
(vi) adding to the sample a 5'-3' DNA polymerase and dNTPs and incubating for filling in of overhanging primer ends;
(vii) inactivation of DNA polymerase by heat treatment of the sample;
(viii) adding to the sample an RNA polymerase which binds to said RNA polymerase promoter site, NTPs and the primer of step (v), and incubating for in vitro transcription of said DNA;
(ix) adding to the sample a reverse transcriptase, an RNAse, dNTPs and the primer of step (v) and incubating reverse transcribing the RNA products of step (viii) to create single-stranded DNA products;
(x) adding to the sample an RNAse, a DNA polymerase and dNTPs for second strand synthesis of the single-stranded DNA of step (ix);
(xi) optionally, repeating steps (viii)-(x).

3. A method according to claim 1 or claim 2, wherein said primers further comprise a restriction enzyme cleavage site downstream of the RNA polymerase promoter site sequence.

4. A method according to claim 3, further comprising the step of removing the primers from the DNA ends by digestion with a restriction enzyme that recognizes said restriction enzyme cleavage site.

5. A method according to any one of claims 1 to 4, wherein said 5'-3' DNA polymerase used to synthesise DNA complementary to the primer overhangs is a Klenow polymerase.

6. A method according to any one of claims 1 to 5, wherein said RNA polymerase is a T7 RNA polymerase

7. A method according to any one of claims 1 to 6, wherein said reverse transcription is carried out using AMV reverse transcriptase.

8. A method according to any one of claims 1 to 7, wherein said second strand synthesis is carried out using Taq polymerase

9. A method according to any one of claims 1 to 8, wherein said primers further comprise a restriction enzyme cleavage site downstream of the RNA polymerase promoter site and upstream of the poly A tail, wherein said restriction enzyme site is optionally a Bpm1 site, and wherein said poly A tail is optionally 15 nucleotides in length.

10. A method according to any one of claims 1 to 9, wherein all the steps of said method up to and including the reverse transcriptase step may be carried out in the same vessel..

11. A method according to any one of claims 1 to 10, wherein one or more of the steps of said method are carried out in a buffer comprising 20mM Tris-acetate, 10mM magnesium acetate, 50mM potassium acetate, and 1mM dithiothreitol at pH 7.9, and optionally wherein all of the steps of said method are carried out in said buffer.

12. A method according to any one of claims 1 to 11, further comprising sequencing of the amplified DNA fragments, optionally by high-throughput sequencing.

13. A method according to any one of claims 1 to 12, wherein said sample of double stranded DNA fragments is obtained by ChIP, reCHiP, ChIA-PET or Hi-C.

14. A method according to any one of claims 1 to 13, comprising the steps
(i) incubating the double-stranded DNA sample with alkaline phosphatase at 37°C in order to dephosphorylate 3' ends;
(ii) inactivation of alkaline phosphatase by heat treatment of the sample;
(iii) incubating the sample with terminal transferase and dTTPs at 37°C for T-tailing of DNA ends;
(iv) inactivation of terminal transferase by heat treatment of the sample;
(v) allowing re-annealing of the sample DNA and adding to the sample primers comprising an T7 RNA polymerase promoter site upstream of a poly-A tail, and incubating at 37°C to allow annealing of the primers to the sample DNA;
(vi) adding to the sample Klenow polymerase and dNTPs and incubating at 37°C;
(vii) inactivation of Klenow polymerase and denaturation of DNA fragments to create single-stranded DNA by heat treatment of the sample;
(viii) in vitro transcribing said DNA using T7 RNA polymerase;
(ix) extracting the RNA products of step (iv);
(x) reverse transcribing the RNA products of step (iv) to create single-stranded DNA products;
(xi) incubating with Taq polymerase, Pfu polymerase and RNAse H at 37°C to creating double stranded DNA fragments by second strand synthesis of the single-stranded DNA of step (ix) ;
(xii) optionally, repeating steps (viii)-(xi);
wherein steps (i) - (viii) of said method are carried out in a buffer comprising 20mM Tris-acetate, 10mM magnesium acetate, 50mM potassium acetate, 1 mM dithiothreitol at pH 7.9, and optionally wherein steps (i) - (viii) of said method are carried out in a single reaction vessel.

## Patentansprüche

1. Verfahren zur linearen DNA-Amplifikation, umfassend die Schritte:
(i) T-tailing der DNA-Enden von doppelsträngigen DNA-Fragmenten in einer Probe;
(ii) Binden von Primern, die eine RNA-Polymerase-Promoterstelle stromaufwärts eines Poly-A-Schwanzes aufweisen, an besagte Fragmente, wobei besagte Primer an die Poly-T-Enden besagter Fragmente binden;
(iii) Verwenden einer 5'-3'-DNA-Polymerase zur Synthetisierung von DNA, die zu den Primerüberhängen komplementär ist, um doppelsträngige DNA-Fragmente mit einer RNA-Polymerase-Promoterstelle an beiden Enden zu erzeugen;
(iv) In-vitro-Transkription besagter DNA unter Verwendung einer RNA-Polymerase, die an besagte RNA-Polymerase-Promoterstelle bindet;
(v) Reverse Transkription der RNA-Produkte aus Schritt (iv), um einzelsträngige DNA-Produkte zu erzeugen;
(vi) Erzeugen von doppelsträngigen DNA-Fragmenten durch Zweitstrang-Synthese der einzelsträngigen DNA aus Schritt (v);
(vii) optional, Wiederholen der Schritte (iv)-(vi).

2. Verfahren gemäß Anspruch 1, umfassend die Schritte:
(i) Inkubieren der doppelsträngigen DNA-Probe mit alkaliner Phosphatase, um 3'-Enden zu dephosphorylieren;
(ii) Inaktivierung der alkalinen Phosphatase durch Hitzebehandlung der Probe;
(iii) Zugeben einer terminalen Transferase und dTTPs zu der Probe und Inkubieren für ein T-tailing der DNA-Enden;
(iv) Inaktivierung der terminalen Transferase durch Hitzebehandlung der Probe;
(v) Zugeben von Primern, die eine RNA-Polymerase-Promoterstelle stromaufwärts eines Poly-A-Schwanzes enthalten zu der Probe und Inkubieren, um das Binden der Primer zur Proben-DNA zu erlauben;
(vi) Zugeben einer 5'-3'-DNA-Polymerase und dNTPs zu der Probe und Inkubieren, um überhängende Primerenden aufzufüllen;
(vii) Inaktivierung der DNA-Polymerase durch Hitzebehandlung der Probe;
(viii)Zugeben einer RNA-Polymerase, die an besagte RNA-Polymerase-Promoterstelle bindet, NTPs und des Primers aus Schritt (v) zu der Probe, und Inkubieren zur in-vitro-Transkription besagter DNA;
(ix) Zugeben einer reversen Transkriptase, einer RNAse, dNTPs und des Primers aus Schritt (v) zu der Probe und Inkubieren, um die RNA-Produkte aus Schritt (viii) revers zu transkribieren und einzelsträngige DNA-Produkte zu erstellen;
(x) Zugeben einer RNAse, einer DNA-Polymerase und dNTPs zu der Probe für die Zweitstrang-Synthese der einzelstränigen DNA aus Schritt (ix);
(xi) optional, Wiederholen der Schritte (viii)-(x).

3. Verfahren gemäß Anspruch 1 oder Anspruch 2, wobei besagte Primer stromabwärts der RNA-Polymerase-Promoterstellensequenz ferner eine Restriktionsenzymschnittstelle enthalten.

4. Verfahren gemäß Anspruch 3, ferner enthaltend den Schritt des Entfernens der Primer von den DNA-Enden durch Verdau mit einem Restriktionsenzym, das besagte Restriktionsenzymschnittstelle erkennt.

5. Verfahren gemäß einem der Ansprüche 1 bis 4, wobei besagte 5'-3'-DNA-Polymerase, die zur Synthese der zu den Primerüberhängen komplementären DNA verwendet wird, eine Klenow-Polymerase ist.

6. Verfahren gemäß einem der Ansprüche 1 bis 5, wobei besagte RNA-Polymerase eine T7-RNA-Polymerase ist.

7. Verfahren gemäß einem der Ansprüche 1 bis 6, wobei besagte reverse Transkription unter Verwendung der reversen Transkriptase AMV durchgeführt wird.

8. Verfahren gemäß einem der Ansprüche bis 7, wobei besagte Zweitstrang-Synthese unter Verwendung der Taq-Polymerase durchgeführt wird.

9. Verfahren gemäß einem der Ansprüche 1 bis 8, wobei besagte Primer ferner eine Restriktionsenzymschnittstelle stromabwärts der RNA-Polymerase-Promoterstelle und stromaufwärts des Poly-A-Schwanzes enthalten, wobei besagte Restriktionsenzymstelle optional eine Bpm1 Stelle ist, und wobei besagter Poly-A-Schwanz optional 15 Nukleotide lang ist.

10. Verfahren gemäß einem der Ansprüche 1 bis 9, wobei alle Schritte von besagem Verfahren bis zu und einschließlich des Schrittes der reversen Transkription in demselben Behälter ausgeführt werden können.

11. Verfahren gemäß einem der Ansprüche 1 bis 10, wobei einer oder mehrere Schritt von besagtem Verfahren in einem Puffer durchgeführt werden, der 20 mM Tris-acetat, 10 mM Magnesiumacetat, 50 mM Kaliumacetat, und 1 mM Dithiothreitol bei pH 7.9 enthält, und wobei optional alle Schritte von besagtem Verfahren in besagtem Puffer durchgeführt werden.

12. Verfahren gemäß einem der Ansprüche 1 bis 11, ferner umfassend die Sequenzierung der amplifizierten DNA-Fragmente, optional durch Hochdurchsatz-Sequenzierung.

13. Verfahren gemäß einem der Ansprüche 1 bis 12, wobei besagte Probe doppelsträngiger DNA-Fragmente durch ChIP, reCHiP, ChIA-PET oder Hi-C erhalten wird.

14. Verfahren gemäß einem der Ansprüche 1 bis 13, umfassend die Schritte
(i) Inkubieren der doppelsträngigen DNA-Probe mit alkaliner Phosphatase bei 37°C, um 3'-Enden zu dephosphorylieren;
(ii) Inaktivierung der alkalinen Phosphatase durch Hitzebehandlung der Probe;
(iii) Inkubieren der Probe mit terminaler Transferase und dTTPs bei 37°C für das T-tailing der DNA-Enden;
(iv) Inaktivierung der terminalen Transferase durch Hitzebehandlung der Probe;
(v) Erlauben der Wieder-Bindung der Proben-DNA und Zugeben von Primern, die eine T7-RNA-Polymerase-Promoterstelle stromaufwärts eines Poly-A-Schwanzes aufweisen, zur Probe und Inkubieren bei 37°C, um die Bindung der Primer an die Proben-DNA zu erlauben;
(vi) Zugeben von Klenow-Polymerase und dNTPs zur Probe und Inkubieren bei 37°C;
(vii) Inaktivierung der Klenow-Polymerase und Denaturierung der DNA-Fragmente durch Hitzebehandlung der Probe, um einzelsträngige DNA zu erstellen;
(viii)In-vitro-Transkription besagter DNA unter Verwendung von T7-RNA-Polymerase;
(ix) Extrahieren der DNA-Produkte aus Schritt (iv);
(x) Reverse Transkription der RNA-Produkte aus Schritt (iv), um einzelsträngige DNA-Produkte zu erstellen;
(xi) Inkubieren mit Taq-Polymerase, Pfu-Polymerase und RNAse H bei 37°C, um doppelsträngige DNA-Fragmente durch Zweitstrang-Synthese der einzelsträngigen DNA aus Schritt (ix) zu erstellen;
(xii) optional, Wiederholen der Schritte (viii)-(xi);
wobei die Schritte (i) - (viii) von besagtem Verfahren in einem Puffer durchgeführt werden, der 20 mM Tris-acetat, 10 mM Magnesiumacetat, 50 mM Kaliumacetat, 1 mM Dithiothreitol bei pH 7.9 enthält, und wobei optional die Schritte (i) - (viii) von besagtem Verfahren in einem einzelnen Reaktionsbehälter durchgeführt werden.

## Revendications

1. Procédé d'amplification d'ADN linéaire comprenant les étapes qui consistent à:
(i) ajouter une queue poly-T aux extrémités d'ADN de fragments d'ADN double brin dans un échantillon ;
(ii) anneler auxdits fragments des amorces comprenant un site promoteur d'ARN polymérase en amont d'une queue poly-A, où lesdites amorces s'annèlent aux extrémités poly-T desdits fragments ;
(iii) utiliser une ADN polymérase 5'-3' pour synthétiser un ADN complémentaire aux surplombs des amorces, afin de créer des fragments d'ADN double brin avec un site promoteur d'ARN polymérase aux deux extrémités ;
(iv) transcrire *in vitro* ledit ADN en utilisant une ARN polymérase qui se lie audit site promoteur d'ARN polymérase ;
(v) réaliser une transcription inverse des produits d'ARN de l'étape (iv) afin de créer des produits d'ADN simple brin ;
(vi) créer des fragments d'ADN double brin par une synthèse du second brin de l'ADN simple brin de l'étape (v) ;
(vii) facultativement, répéter les étapes (iv)-(vi).

2. Procédé selon la revendication 1, comprenant les étapes qui consistent à :
(i) incuber l'échantillon d'ADN double brin avec une phosphatase alcaline afin de déphosphoryler les extrémités 3' ;
(ii) inactiver la phosphatase alcaline par un traitement de l'échantillon par la chaleur ;
(iii) ajouter à l'échantillon une transférase terminale et des dTTP et incuber pour ajouter une queue poly-T aux extrémités d'ADN ;
(iv) inactiver la transférase terminale par un traitement de l'échantillon par la chaleur ;
(v) ajouter à l'échantillon des amorces comprenant un site promoteur d'ARN polymérase en amont d'une queue poly-A et incuber pour permettre un annelage des amorces à l'ADN de l'échantillon ;
(vi) ajouter à l'échantillon une ADN polymérase 5'-3' et des dNTP et incuber pour combler les extrémités des amorces en surplomb ;
(vii) inactiver l'ADN polymérase par un traitement thermique de l'échantillon ;
(viii) ajouter à l'échantillon une ARN polymérase qui se lie audit site promoteur d'ARN polymérase, aux NTP et à l'amorce de l'étape (v), et incuber pour permettre une transcription *in vitro* dudit ADN ;
(ix) ajouter à l'échantillon une transcriptase inverse, une RNAse, des dNTP et l'amorce de l'étape (v) et incuber pour réaliser une transcription inverse des produits d'ARN de l'étape (viii) afin de créer des produits d'ADN simple brin ;
(x) ajouter à l'échantillon une RNAse, une ADN polymérase et des dNTP pour permettre la synthèse du second brin de l'ADN simple brin de l'étape (ix) ;
(xi) facultativement, répéter les étapes (viii)-(x).

3. Procédé selon les revendications 1 ou 2, dans lequel lesdites amorces comprennent en outre un site de clivage d'enzyme de restriction en aval de la séquence du site promoteur d'ARN polymérase.

4. Procédé selon la revendication 3, comprenant en outre l'étape d'élimination des amorces des extrémités d'ADN par une digestion avec une enzyme de restriction qui reconnaît ledit site de clivage d'enzyme de restriction.

5. Procédé selon l'une quelconque des revendications 1 à 4, dans lequel ladite ADN polymérase 5'-3' utilisée pour synthétiser un ADN complémentaire aux surplombs des amorces est une polymérase Klenow.

6. Procédé selon l'une quelconque des revendications 1 à 5, dans lequel ladite ARN polymérase est une ARN polymérase de T7.

7. Procédé selon l'une quelconque des revendications 1 à 6, dans lequel ladite transcription inverse est réalisée en utilisant une transcriptase inverse AMV.

8. Procédé selon l'une quelconque des revendications 1 à 7, dans lequel ladite synthèse du second brin est réalisée en utilisant une Taq polymérase.

9. Procédé selon l'une quelconque des revendications 1 à 8, dans lequel lesdites amorces comprennent en outre un site de clivage d'enzyme de restriction en aval du site promoteur d'ARN polymérase et en amont de la queue poly-A, où ledit site d'enzyme de restriction est facultativement un site Bpm1, et où ladite queue poly-A possède facultativement une longueur de 15 nucléotides.

10. Procédé selon l'une quelconque des revendications 1 à 9, dans lequel toutes les étapes dudit procédé, jusqu'à et incluant l'étape de transcriptase inverse, peuvent être réalisées dans le même récipient.

11. Procédé selon l'une quelconque des revendications 1 à 10, dans lequel une ou plusieurs des étapes dudit procédé sont réalisées dans un tampon comprenant du Tris-acétate 20 mM, de l'acétate de magnésium 10 mM, de l'acétate de potassium 50 mM, et du dithiothréitol 1 mM à un pH 7,9, et facultativement dans lequel toutes les étapes dudit procédé sont réalisées dans ledit tampon.

12. Procédé selon l'une quelconque des revendications 1 à 11, comprenant en outre le séquençage des fragments d'ADN amplifiés, facultativement par un séquençage à haut débit.

13. Procédé selon l'une quelconque des revendications 1 à 12, dans lequel ledit échantillon de fragments d'ADN double brin est obtenu par ChIP, reCHiP, ChIA-PET ou Hi-C.

14. Procédé selon l'une quelconque des revendications 1 à 13, comprenant les étapes qui consistent à
(i) incuber l'échantillon d'ADN double brin avec une phosphatase alcaline à 37 °C afin de déphosphoryler les extrémités 3' ;
(ii) inactiver la phosphatase alcaline par un traitement thermique de l'échantillon ;
(iii) incuber l'échantillon avec une transférase terminale et des dTTP à 37 °C pour ajouter une queue poly-T aux extrémités d'ADN ;
(iv) inactiver la transférase terminale par un traitement thermique de l'échantillon ;
(v) permettre un re-annelage de l'ADN de l'échantillon et ajouter à l'échantillon des amorces comprenant un site promoteur d'ARN polymérase de T7 en amont d'une queue poly-A, et incuber à 37 °C pour permettre un annelage des amorces à l'ADN de l'échantillon ;
(vi) ajouter à l'échantillon une polymérase Klenow et des dNTP et incuber à 37 °C ;
(vii) inactiver la polymérase Klenow et dénaturer les fragments d'ADN afin de créer un ADN simple brin par un traitement thermique de l'échantillon ;
(viii) transcrire *in vitro* ledit ADN en utilisant une ARN polymérase de T7 ;
(ix) extraire les produits d'ARN de l'étape (iv) ;
(x) réaliser une transcription inverse des produits d'ARN de l'étape (iv) afin de créer des produits d'ADN simple brin ;
(xi) incuber avec une Taq polymérase, une Pfu polymérase et une RNAse H à 37 °C afin de créer des fragments d'ADN double brin par la synthèse du second brin de l'ADN simple brin de l'étape (ix) ;
(xii) facultativement, répéter les étapes (viii)-(xi) ;
où les étapes (i)-(viii) dudit procédé sont réalisées dans un tampon comprenant 20 mM de Tris-acétate, 10 mM de l'acétate de magnésium, 50 mM d'acétate de potassium , et 1 mM de dithiothréitol ayant un pH de 7,9, et facultativement où les étapes (i)-(viii) dudit procédé sont réalisées dans un seul récipient de réaction.


### SEQUENCE LISTING

<110> INSERM
<120> Linear DNA amplification
<130> BET12P0762
<150> EP 11305531.3
   <151> 2011-05-05
<160> 3
<170> PatentIn version 3.5
<210> 1
   <211> 23
   <212> DNA
   <213> Artificial
<220>
   <223> T7 promoter from bacteriophage T7
<400> 1
   taatacgact cactataggg aga 23
<210> 2
   <211> 18
   <212> DNA
   <213> Artificial
<220>
   <223> SP6 promoter from bacteriophage SP6
<400> 2
   atttaggtga cactatag 18
<210> 3
   <211> 20
   <212> DNA
   <213> Artificial
<220>
   <223> T3 promoter from bacteriophage T3
<400> 3
   attaaccctc actaaaggga 20